(19) Europäisches Patentamt
European Patent Office
Office européen des brevets

(11) **EP 3 365 155 B1**

(12) **EUROPÄISCHE PATENTSCHRIFT**

(45) Veröffentlichungstag und Bekanntmachung des Hinweises auf die Patenterteilung:
**07.04.2021   Patentblatt 2021/14**

(21) Anmeldenummer: **16791543.8**

(22) Anmeldetag: **24.10.2016**

(51) Int Cl.:
**B29C 64/106** (2017.01)

(86) Internationale Anmeldenummer:
**PCT/EP2016/075514**

(87) Internationale Veröffentlichungsnummer:
**WO 2017/068177 (27.04.2017 Gazette 2017/17)**

(54) **ELEKTROSTRIKTIV UNTERSTÜTZTE, ADDITIVE FERTIGUNG PHYSISCHER OBJEKTE UND ENTSPRECHENDE VORRICHTUNG**

ELECTROSTRICTIVELY ASSISTED ADDITIVE MANUFACTURE OF PHYSICAL OBJECTS AND CORRESPONDING APPARATUS

FABRICATION ADDITIVE, ASSISTÉE PAR ÉLECTROSTRICTION D'OBJETS PHYSIQUES AND APPAREIL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.10.2015   DE 102015013680**

(43) Veröffentlichungstag der Anmeldung:
**29.08.2018   Patentblatt 2018/35**

(73) Patentinhaber: **Meier, Bernd
64285 Darmstadt (DE)**

(72) Erfinder:
• **JANKOWIAK-MEIER, Iris Theresia
64285 Darmstadt (DE)**
• **MEIER, Clara
64285 Darmstadt (DE)**
• **FREUND, Nele
64287 Darmstadt (DE)**
• **MEIER, Bernd
64285 Darmstadt (DE)**

(74) Vertreter: **Habermann Intellectual Property Partnerschaft von Patentanwälten mbB
Dolivostraße 15A
64293 Darmstadt (DE)**

(56) Entgegenhaltungen:
**US-A1- 2004 251 581     US-A1- 2011 136 162
US-A1- 2012 018 924**

**Beschreibung**

Die vorliegende Erfindung betrifft ein Verfahren zur elektrostriktiv unterstützten, additiven Fertigung eines räumlich ausgebildeten physischen Objekts sowie eine Vorrichtung zur elektrostriktiv unterstützten, additiven Fertigung und Applikation eines räumlich ausgebildeten physischen Objekts.

Die Behandlung des menschlichen Körpers mit hochfrequenten Wechselströmen ist als chirurgisches Verfahren zur Blutstillung und zur Koagulation bei operativen Eingriffen die Blutung von Gewebe bekannt. Die dabei angewendeten Frequenzen sind so hoch gewählt, dass Nerven, Muskeln und insbesondere das Herz nicht zu unkontrollierten Aktionspotentialen im Sinne von tetanischen Kontraktionen und Rhythmusstörungen angeregt werden. Die Energie der applizierten elektrischen Felder wird dabei derart angepasst, dass die Gewebeschäden nur in denjenigen Bereichen auftreten, die koaguliert bzw. kauterisiert werden sollen. Die Elektrokauterisation wird sowohl bei der konventionell chirurgischen Schnitteröffnung von Körperhöhlen, z.B. Laparotomien, aber auch bei endoskopischen Eingriffen insbesondere zur Blutstillung, Koagulation und Entfernung von Gewebestrukturen verwendet.

Bei der Blutstillung mittels Elektrokauterisation kommt es zu Verletzungen des die Blutungsquelle umgebenden Gewebes, d.h. die Gewebestruktur von im Körper verbleibenden und von entnommenem Gewebe wird irreversibel gestört. Zudem besteht die Gefahr, dass Nervengewebe und Reizleitungssystem des Herzens bereits durch schwache Wechselströme empfindlich beeinträchtigt werden können.

Blutungen aus parenchymatösen Organen wie Leber, Nieren oder Milz können mittels Elektrokauterisation nur unbefriedigend gestillt werden, da das koagulierte Gewebe immer wieder aufbricht.

Aus der US 2004/0251581 A1 sind ein Verfahren und eine Vorrichtung zur Herstellung von Objekten im Mikron- oder Nanometerbereich auf einer Zieloberfläche bekannt. Dabei werden ausgewählte Fluidphasenzusammensetzungen in eine Phasenänderungskammer eingeführt, die eine Abflussöffnung in Richtung der Zieloberfläche aufweist. In der Phasenänderungskammer wird eine fokussierte Plasmaentladungsquelle betrieben. Die Elektroden der Plasmaentladungsquelle sind im Inneren der Phasenänderungskammer angeordnet. Das abscheidbare Material entsteht aufgrund der Einwirkung des Plasmas auf die Fluidzusammensetzungen durch eine chemische Reaktion und/oder einen physikalischen Übergang im Inneren der Phasenänderungskammer, passiert eine Öffnung der Kammer und wird auf der Zieloberfläche in der Nähe der Phasenänderungskammer abgeschieden.

Aus der US 2011/0136162 A1 ist ein Mikroplasmasystem zur funktionalisierten Strukturierung eines Gewebereekonstruktionssubstrats bekannt. Das System umfasst eine Mikroplasmadüse, die angrenzend an ein Substratmaterial befestigt ist, das an einer Plattform befestigt ist. Die Plattform kann durch ein Bewegungssteuerungssystem in Bezug auf die feste Mikroplasmadüse bewegt werden, sodass ein funktionalisiertes Muster auf der Oberfläche des Substratmaterials erzeugt werden kann. Das bevorzugte Substratmaterial ist Polycaprolacton. Das Mikroplasmasystem kann Teil eines Materialzuführsystems sein, das mehrere Düsen unterschiedlicher Art umfasst; wobei mindestens eine der Düsen mindestens ein Substratmaterial abscheidet, und mindestens eine der Düsen mindestens einen Zelltyp abscheidet, und mindestens eine der Düsen mindestens ein Biomolekül abscheidet, wodurch ein Gerüst mit einem funktionalisierten Mikroplasma-Muster konstruiert werden kann.

Aus der US 2012/0018924 A1 ist ein Verfahren zum Aufbau eines dreidimensionalen Modells mit einem extrusionsbasierten additiven Fertigungssystem bekannt. Das Fertigungssystem weist eine Verflüssigungsanordnung mit einem Verflüssigungsrohr und einer am unteren Ende des Verflüssigungsrohrs montierten Extrusionsspitze auf. Entlang des Verflüssigungsrohres sind unabhängig voneinander beheizbare Zonen angeordnet. Ein thermoplastisches Material wird innerhalb der ersten Zone des Verflüssigungsrohrs zumindest teilweise geschmolzen. Das geschmolzene thermoplastische Material wird aus der Extrusionsspitze extrudiert.

Demgegenüber liegt der vorliegenden Erfindung die Aufgabe zugrunde, ein Verfahren und eine Vorrichtung zur additiven Fertigung eines räumlich ausgebildeten physischen Objekts an einem vorbestimmten Bildungsort zu schaffen, welche einen kontrollierbaren Aufbau von Gewebeersatzobjekten ermöglichen und die insbesondere für den direkten Einsatz bei operativen Eingriffen am menschlichen oder tierischen Körper geeignet sind.

Diese Aufgabe wird mit einem Verfahren mit den Merkmalen des Anspruchs 1 und einer Vorrichtung mit den Merkmalen des Anspruchs 8 gelöst. Weitere vorteilhafte Ausgestaltungen sind jeweils in den Unteransprüchen beschrieben.

Bei Versuchen mit hochfrequenten Wechselströmen hatte sich überraschend gezeigt, dass deren Wirkung nicht nur zur Elektrokauterisation von Gewebe genutzt werden kann, sondern dass durch die energetische Wirkung hochfrequenter Wechselstromfelder polymere Dielektrika miteinander chemisch verbunden werden können.

Das erfindungsgemäße Verfahren ermöglicht demnach die generative Fertigung eines physischen Objekts durch gezielte Platzierung und kovalente Vernetzung von Polymeren in einem elektrischen Wechselstromfeld mit wählbarer Position, Form, Spannung, Frequenz und Stromstärke.

Mit dem erfindungsgemäßen Verfahren eröffnet sich die Möglichkeit, ein räumlich ausgebildetes physisches Objekt in der Art des 3D-Drucks an einem vorbestimmten Bildungsort additiv zu fertigen. Besonders vorteilhaft kann das erfindungsgemäße Verfahren im Zuge eines operativen Eingriffs am menschlichen oder tierischen Körper direkt im oder an dem menschlichen oder tierischen Körper durchgeführt werden. Das physische Objekt kann beispielsweise als Matrix oder Depotstruktur für arzneilich wirksame Stoffe oder Farbmarker, Gewebeschutzabdeckung bei Organdefekten und Verletzungen, Deck- oder Füllkörper in der plastisch-ästhetischen Chirurgie, Stütz- oder Bindegewebe für Knorpel oder Knochen, Knochenersatz, Ersatz für Körpergewebe oder sonstiges Hilfsgewebe, z.B. zum Stillen von Blutungen etc. im menschlichen oder tierischen Körper eingesetzt werden. Es versteht sich jedoch, dass im Rahmen der Erfindung der Bildungsort für das physische Objekt auch außerhalb des menschlichen oder tierischen Körpers vorgesehen sein kann.

Erfindungsgemäß wird das physische Objekt als Vernetzungsprodukt durch eine elektrostriktiv unterstützte chemische Reaktion in mindestens einem elektrischen Wechselstromfeld aus einem ersten Polymer mit ersten reaktiven Seitengruppen und einem zweiten Polymer mit zweiten reaktiven Seitengruppen erzeugt. Die ersten und zweiten Polymere werden dabei als Dielektrika in dem Wechselstromfeld energetisch angeregt, sodass eine Vernetzung erfolgt.

Im Rahmen der Erfindung umfasst die energetische Wirkung des Wechselstromfeldes insbesondere die elektrostriktive Deformation, den invers piezoelektrischen Effekt, den dielektrischen Verlust, eine Radikalbildung sowie Polarisationsbewegungen geladener Gruppen.

Unter Vernetzung wird im Rahmen der Erfindung die Bildung kovalenter Bindungen, die Bildung von Ionenbindungen, die Bildung von Van-der Waals-Wechselwirkungen und die Bildung von interpenetrierenden Netzwerken verstanden.

Grundsätzlich sind alle bekannten Polymertypen, deren Monomere zumindest zum Teil reaktive Seitengruppen aufweisen, für eine elektrostriktiv und energetisch induzierte chemische Vernetzung geeignet. Das erste und das zweite Polymer können dabei gleich oder unterschiedlich oder jeweils selbst Polymergemische sein. Beim Einsatz des erfindungsgemäßen Verfahrens am menschlichen oder tierischen Körper steht das gebildete physische Objekt in einem steten Kontakt zu den Körpergeweben, sodass das körperliche Abwehrsystem das physische Objekt als Fremdkörper erkennt und gegebenenfalls eine Abwehrreaktion auslöst, die zur Abstoßung des physischen Objekts und im ungünstigen Fall zu Entzündungsreaktionen führen kann. Aus diesem Gründen ist es besonders vorteilhaft, in dem erfindungsgemäßen Verfahren erste und zweite Polymere einzusetzen, die keine oder nur eine geringfügige Immunreaktionen hervorrufen und die gegebenenfalls biologisch abbaubar sind.

Derartige immunologisch gut verträgliche Polymere sind bekannt. Erfindungsgemäß besonders geeignet sind Polysaccharide, insbesondere überwiegend α-1,4-glycosidisch verknüpfte Polysaccharide (z.B. Stärke und deren Derivate, Pektin) und überwiegend β-1,4-glycosidisch verknüpfte Polysaccharide (z.B. Cellulose und deren Derivate, Chitosan und Chitin) sowie Proteine. Die Verwendung derartiger gut verträglicher Polymere hat in vielen Fällen zudem den Vorteil, dass die mit dem erfindungsgemäßen Verfahren erzeugten physischen Objekte biologisch abbaubar sind.

Die in dem erfindungsgemäßen Verfahren eingesetzten ersten und zweiten Polymere weisen reaktive Seitengruppen auf, die für die Bildung einer Netzwerkstruktur geeignet sind. Die Monomer-Einheiten, aus denen die ersten und zweiten Polymere bestehen, sind dabei teilweise oder vollständig mit einer oder mehreren reaktiven Seitengruppen substituiert. Vorzugsweise weisen die reaktiven Seitengruppen Reste auf, die aus der Gruppe bestehend aus Carboxyl- (-COOH), Hydroxyl-(-OH), Aldehyd (-CHO,) Amino-(-NH$_2$), Carbonsäureester (-COOAlk, wobei Alk eine Alkylgruppe mit 1 bis 9 Kohlenstoffatomen, insbesondere 1 bis 4 Kohlenstoffatomen ist), Carbonsäureamid, Sulfonsäure, Sulfonsäureamid und Hydrogensulfat sowie Mischungen hiervon ausgewählt sind.

Besonders bevorzugt sind die Reste der reaktiven Seitengruppen ausgewählt aus der Gruppe bestehend aus Carboxymethyl, Carboxyethyl, Hydroxyethyl, Hydroxymethyl, Amino, Aldehyd, Carbonsäure, Carbonsäureamid, Sulfonamid, Carbonsäuresalz, Sulfonsäuresalz, Schwefelsäure, Sulfat, Hydrogensulfat und Schwefelsäureamid sowie Mischungen daraus.

Geeignete erste und zweite Polymere, die für den Einsatz in dem erfindungsgemäßen Verfahren bei Anwendung am menschlichen oder tierischen Körper verwendet werden können, sind bevorzugt jeweils unabhängig voneinander ausgewählt aus der Gruppe der gegebenenfalls derivatisierten Polysaccharide bestehend aus Amylosen, Amylopectin, Acemannanen, Arabinogalactanen, Dextranen, Galactomannanen, Alginsäure, Alginsäurederivaten, Alginsäuresalzen, Galactoglucomannanen, Xanthanen, Carrageen, Guaran, Gummiarabikum, Stärken, modifizierte Stärken, Glucosaminoglucanen, Cellulosen, Chitin, Chitosan, Polyuroniden und Pektinen.

Im Hinblick auf die immunologische Verträglichkeit sind die ersten und zweiten Polysaccharide unabhängig voneinander insbesondere ausgewählt aus gegebenenfalls derivatisierten, insbesondere aminierten Polysacchariden, ganz besonders gegebenenfalls aminierte Cellulose- oder Stärkederivate aus der Gruppe bestehend aus Hydroxyalkylstärken, insbesondere Hydroxyethylstärken, Carboxyalkylstärken, insbesondere Carboxymethylstärken, Hydroxyalkyl-Carboxyalkylstärken, insbesondere Hydroxyethyl-Carboxymethylstärken, veresterte Stärken, Alkylester von Carboxyalkylstärken, Carboxyalkylcellulose, insbesondere Carboxymethylcellulose, Hydroxyethylcellulose, Carboxymethyl-hydroxyethylcellulose, Alkylester der Carboxyalkylcellulose, Polyuronide, Alkylketten, insbesondere C$_1$- bis C$_4$-Alkyl-

ketten bezeichnet.

In einer bevorzugten Ausgestaltung der vorliegenden Erfindung weist die reaktive Seitengruppe des ersten oder das zweiten Polysaccharid wenigstens eine natürlich vorhandene oder chemisch eingeführte Aminogruppe, bevorzugt eine primäre Aminogruppe auf. Eine Aminogruppe kann in bekannter Weise beispielsweise durch reduktive Aminierung in die Monomereinheiten eines Polysaccharids eingeführt werden. Dem Fachmann dem sind reduktive Aminierungen mit Ammoniak, Alkylaminen, Dialkylaminen, Ammoniumhydroxid in Anwesenheit eines Redoxkatalysators wie z.B. Raney-Nickel bekannt. Die reduktive Aminierung von Hydroxyalkyl-Celluloseverbindungen ist beispielsweise in der US 4124758 A beschrieben. Für die Verwendung als erstes oder zweites Polymer bevorzugt sind Aminogruppen enthaltende Polysaccharide ausgewählt aus der Gruppe bestehend aus Chitin, Chitosan, aminierter Hydroxyethylstärke, aminierter Carboxymethylstärke, aminierter Carboxyethylstärke, aminierter Hydroxyethyl-carboxymethylstärke, aminierter Hydroxyalkylstärke und aminierten Viskosefasern.

In einer besonders bevorzugten Ausgestaltung der vorliegenden Erfindung weist die reaktive Seitengruppe des ersten oder des zweiten Polysaccharids wenigstens eine Aldehydgruppe auf. Die Einführung einer oder mehrerer Aldehydgruppen in die Monomer-Einheiten eines Polysaccharids kann vorab chemisch durch Umsetzung mit einem Oxidationsmittel erfolgen. Die Oxidation von Polysacchariden mit Natriumperiodat ist Fachleuten bekannt. Beispielsweise ist die Oxidation von Cellulose zu einer Cellulose-2,3-dialdehydverbindung in Lindh. J., Carlson, O.C., Stromme, M. und Mihranan, A. "Convenient One-Pot Formation of 2,3-Dialdehyd-Cellulose Beads via Peroxidate Oxidation of Cellulose in Water", Biomacromolecules, 15:1928-1932 (2014) beschrieben. Vorteilhafterweise kann anstelle von Cellulose auch eine Carboxyalkylcellulose eingesetzt werden, wobei Alkyl jeweils $C_1$- bis $C_9$-Alkylketten, insbesondere $C_1$- bis $C_4$-Alkylketten bezeichnet.

In einer weiteren vorteilhaften Ausgestaltung können Viskosefasern zu Dialdehydviskose oxidiert und als erstes oder zweites Polymer eingesetzt werden.

In einer besonders bevorzugten Ausgestaltung der Erfindung ist das erste Polymer ein Polysaccharid, das Monomere aufweist, deren Seitengruppen wenigstens einen Aldehydrest aufweisen, z.B. 2,3-Dialdehyd-carboxyalkylcellulose, wobei die Alkylgruppe 1 bis 9 Kohlenstoffatome, insbesondere 1 bis 4 Kohlenstoffatome aufweist, und das zweite Polymer ist ein Polysaccharid, das Monomere aufweist, deren Seitengruppen wenigstens eine primäre Aminogruppe aufweisen, z.B. Chitin, Chitosan und Aminoderivate davon. Cellulosederivate und Chitin-/Chitosanverbindungen eignen sich im besonderen Maße für den Einsatz in dem erfindungsgemäßen Verfahren zur Herstellung von physischen Objekten, die dazu bestimmt sind, mit dem menschlichen oder tierischen Körper in längerfristigem Kontakt zu stehen, da durch Cellulosederivate und Chitin-/Chitosanverbindungen ausgelöste allergische Reaktionen sehr selten auftreten. Es wird davon ausgegangen, dass sich die Immunsysteme aller höheren Organismen sehr früh an die ständige Exposition mit Cellulosederivaten und Chitin-/Chitosanverbindungen anpassen, da diese Stoffe die häufigsten in unserer Umwelt vorkommenden biologischen Polymere sind.

Cellulose ist aus β-1,4-glycosidisch verbundenen Glucose-Einheiten aufgebaut. Die Cellulose ist als unverzweigtes Molekül mit etwa 1000 Cellubiose-Einheiten in der überwiegenden Zahl der organischen Lösemittel nicht löslich. Eine Wasserlöslichkeit der Cellulose kann durch die Einbindung von Carbonsäuren über eine Etherbindung erreicht werden. Für das erfindungsgemäße Verfahren sind Carboxyalkylcellulose und Carboxyalkylcellulosederivate mit 1 bis 9 Kohlenstoffatomen, insbesondere 1 bis 4 Kohlenstoffatomen in dem Alkylrest besonders geeignet. Am meisten bevorzugt ist Carboxymethylcellulose. Für eine endoskopische Applikation sind darüber hinaus Cellulosederivate besonders bevorzugt, die einen Substituenten ausgewählt aus der Gruppe bestehend aus Benzoesäure, Buttersäure, Acrylsäure, Salicylsäure, Valeriansäure, Isovaleriansäure, Arginin, Lysin, Histidin sowie Alkylester mit 1 bis 9 Kohlenstoffatomen, insbesondere 1 bis 4 Kohlenstoffatomen aufweisen.

Erfindungsgemäß versteht man unter Chitin ein lineares Polysaccharid bestehend aus β-1,4-gylcosidisch verknüpften 2-Acetamido-2-desoxy-β-D-glucopyranose-Einheiten, bei denen mindestens etwa 40 % der Acetamidgruppen zu Aminogruppen deacetyliert vorliegen. Unter Chitosan versteht man gemäß der vorliegenden Erfindung ein weitgehend deacetyliertes Derivat des Chitins. Es besteht somit überwiegend aus 2-Amino-2-desoxy-β-D-glucopyranose-Einheiten mit einem geringeren Anteil an 2-Acetamido-2-desoxy-β-D-glucopyranose-Einheiten. Chitin- und Chitosanverbindungen haben den Vorteil, vollständig biologisch abbaubar zu sein. Die Verbindungen werden durch Chitinase, Lysozym und andere lysosomale Enzyme abgebaut. Chitosan ist zudem in verdünnten Säuren, insbesondere biologisch verträglichen verdünnten Säuren, z.B. in verdünnter Essigsäure, löslich und chemisch stabiler als Cellulose. Für den Einsatz in dem erfindungsgemäßen Verfahren ist Chitosan mit einem Deacetylierungsgrad von 60 % bis 90 % bevorzugt und Chitosan mit einem Deacetylierungsgrad von 80 % bis 90 % besonders bevorzugt.

Sofern die erfindungsgemäße, elektrostriktiv unterstützte chemische Reaktion eine Bildung von Iminen (Schiff'schen Basen) umfasst, beispielsweise wenn das erste Polymer erste reaktive Seitengruppen mit wenigstens einem Aldehydrest aufweist und das zweite Polymer zweite reaktive Seitengruppen mit wenigstens einer primären Aminogruppe aufweist, werden in weiterer Ausgestaltung des erfindungsgemäßen Verfahrens in einem anschließenden Reduktionsschritt die in dem gebildeten physischen Objekt entstandenen Imine zu Aminen reduziert, um die Vernetzung zu stabilisieren. Hierzu wird dem gebildeten physischen Objekt ein Reduktionsmittel zugeführt. Als Reduktionsmittel sind beispielsweise Ascorbinsäure oder salzhaltige Hydride wie $LiAlH_4$, $LiBH_4$, $NaBH_4$, $NaHBH_3CN$ geeignet. Das bei dieser Reaktion freiwerdende Wasser kann entfernt, z.B. abgesaugt werden und das physische Objekt gekühlt werden.

Insbesondere wenn das erste Polymer und das zweite Polymer dieselben sind oder dieselben reaktiven Seitengruppen tragen, ist es von Vorteil, dass in dem erfindungsgemäßen Verfahren weiterhin ein Linker in das elektrische Wechselstromfeld eingebracht wird, der in der Lage ist, die ersten reaktiven Seitengruppen des ersten Polymers und die zweiten reaktiven Seitengruppen des zweiten Polymers miteinander zu koppeln.

In einer weiteren vorteilhaften Ausgestaltung des erfindungsgemäßen Verfahrens können somit zusätzliche bi-, tri- oder polyfunktionelle Moleküle als Linker eingesetzt werden, welche die Verknüpfung des ersten Polymers und des zweiten Polymers miteinander sowie gegebenenfalls weiterer Wirkstoffkomponenten und Zusatzpolymere mit dem ersten Polymer und/oder dem zweiten Polymer in dem elektrischen Wechselstromfeld bewirken.

Geeignete Linker sind ausgewählt aus linearen oder verzweigten, gesättigten oder ungesättigten, aliphatischen oder alicyclischen Kohlenwasserstoffmolekülen mit 1 bis 22, vorzugsweise 2 bis 15, besonders bevorzugt 3 bis 8 Kohlenstoffatomen; aromatischen Molekülen, Aryl-$C_1$-$C_4$-Alkanen und Aryl-$C_2$-$C_6$-Alkenen mit 5 bis 12, vorzugsweise 6 bis 12, besonders bevorzugt 6 Kohlenstoffatomen im Arylrest, die gegebenenfalls mit $C_1$-$C_6$-Alkyl- und/oder $C_2$-$C_6$-Alkoxygruppen substituiert sein können; oder Heteroaromaten, Heteroaryl-$C_1$-$C_4$-Alkanen und Heteroaryl-$C_2$-$C_6$-Alkenenen mit 3 bis 8 Kohlenstoffatomen im Heteroarylrest und einem oder zwei Heteroatom(en) ausgewählt aus N, O und S, die mit $C_1$-$C_6$-Alkyl- und/oder $C_2$-$C_6$-Alkoxygruppen substituiert sein können, wobei das Linkermolekül 2 bis 20 funktionelle Gruppen zur Bildung der kovalenten Bindungen enthält, wobei die funktionellen Gruppen jeweils unabhängig voneinander ausgewählt sind aus Hydroxyl- (-OH), Amino- (-$NH_2$), Carboxyl- (-COOH), Isocyanat- (-NCO), Carbonsäurehalogenid- (-C(O)Cl, -C(0)Br und/oder -C(O)I), Carboxylalkylen- (-$(CH_2)_q$-COOH, mit q = 1-10) oder Ester-Gruppen (-COOAlk, wobei Alk eine Alkylgruppe mit 1 bis 9 Kohlenstoffatomen, insbesondere 1 bis 4 Kohlenstoffatomen ist).

In einer bevorzugten Ausführungsform ist der Linker ein bifunktionelles Molekül. Ebenfalls bevorzugt ist der Linker ein trifunktionelles Molekül. Als Linker besonders bevorzugt sind bifunktionelle und trifunktionelle Moleküle, die identische funktionelle Gruppen aufweisen.

Besonders geeignete Linker sind z. B. Dicarbonsäuren, wie Oxalsäure, Malonsäure, Bernsteinsäure, Glutarsäure, Adipinsäure, Pimelinsäure, Azelainsäure, Sebacinsäure, Maleinsäure, Fumarsäure, Sorbinsäure, Phthalsäure, Terephthalsäure, Isophthalsäure, Agaricinsäure, Citronensäure, sowie deren $C_1$-$C_{10}$-Alkylester, Halogenide, und Anhydride; Dialdehyde, wie z.B. Phthalaldehyd, Glutardialdehyd, Succindialdehyd, Malondialdehyd, Glyoxal; Diisocyanate, wie z.B. Toluylendiisocyanat, Bitoluylendiisocyanat, Dianisidindiisocyanat, Tetramethylendiisocyanat, Hexamethylendiisocyanat, m-Phenylendiisocyanat, m-Xylylendiisocyanat, $C_1$-$C_6$-Alkylbenzoldiisocyanat, 1-Chlorbenzol-2,4-diisocyanat, Cyclohexylmethandiisocyanat, 3,3'-Dimethoxydiphenylmethan-4,4'-diisocyanat, 1-Nitrobenzol-2,4-diisocyanat, 1-Alkoxybenzol-2,4-diisocyanat, Ethylendiisocyanat, Propylendiisocyanat, Cyclohexylen-1,2-diisocyanat, 3,3'-Dichloro-4,4'-biphenylendiisocyanat, Diphenylendiisocyanat, 2-Chlorotrimethylendiisocyanat, Butylen-1,2-diisocyanat, Ethylidendiisocyanat, Diphenylmethan-4,4'-diisocyanat, Diphenylethandiisocyanat, 1,5-Naphthalindiisocyanat, Cyclohexandiisocyanat und Isophorondiisocyanat; Diole, wie z.B. Ethylenglycol, Propylenglycol, Butylenglycol, und Neopentylglycol, Pentandiol-1,5, 3-Methylpentandiol-1,5, Bisphenol A, 1,2-Cyclohexandiol oder 1,4-Cyclohexandiol, Caprolactondiol (Reaktionsprodukt aus Caprolacton und Ethylenglycol), hydroxyalkylierte Bisphenole, Trimethylolpropan, Trimethylolethan, Pentaerythritol, Hexandiol-1,6, Heptandiol-1,7, Octandiol-1,8, Butandiol-1,4, 2-Methyloctandiol-1,8, Nonandiol-1,9, Decandiol-1,10, Cyclohexandimethylol, Di-, Tri- und Tetraethylenglykol, Di, Tri- und Tetrapropylenglykol, Polyethylen- und Polypropylenglykole mit einem mittleren Molekulargewicht von 150 bis 15.000, Trimethylolethan, Trimethylolpropan und Pentaerythrit; und/oder Diamine, wie z.B. 1,2-Diaminoethan, 1,2-Diaminopropan oder 1,3-Diaminopropan, 1,2-, 1,3- oder 1,4-Diaminobutan, 1,5-Diaminopentan, 2,2-Dimethyl-1,3-diaminopropan, Hexamethylendiamin, 1,7-Diaminoheptan, 1,8-Diaminooctan, Trimethyl-1,6-diaminohexan, 1,9-Diaminononan, 1,10- Diaminodecan, 1,12-Diaminododecan, 1,2-Diaminocyclohexan, 1,4- Diaminocyclohexan, 1^-Cyclohexanbis(methylamin), 1,2-Phenylendiamin, 1,3-Phenylendiamin, 1,4-Phenylendiamin, 4,4'-Ethylendianilin, 4,4'-Methylendianilin, 4,4'-Diaminostilben, 4,4'-Thiodianilin, 4-Aminophenyldisulfid, 2,6-Diaminopyridin, 2,3-Diaminopyridin, 3,4-Diaminopyridin, 2,4-Diaminopyrimidin, 4,5-Diaminopyrimidin, 4,6-Diaminopyrimidin. Diese Linker sind aufgrund ihrer überwiegend hohen Toxizität vor allem für den Einsatz des erfindungsgemäßen Verfahrens an Bildungsorten außerhalb des menschlichen und tierischen Körpers geeignet.

In einer ganz besonders vorteilhaften Ausführungsform ist der Linker ein bioverträgliches Molekül, insbesondere ein Molekül ausgewählt aus der Gruppe bestehend aus Isoprenoiden mit einem Cyclopentanpyran-Gerüst und mindestens zwei Sauerstoffatomen. Diese sogenannten Iridoide bzw. deren Glycoside werden in Pflanzen als Wehrstoffe gegen Schädlinge synthetisiert. Iridoide haben bekanntermaßen die Eigenschaft u.a. Proteine irreversibel zu vernetzen.

Ein erfindungsgemäß besonders bevorzugter Linker aus der Gruppe der Iridoide ist Genipin. Genipin ist das Aglycon des Geniposids, das aus den Früchten von Gardenia jasminoides Ellis gewonnen wird und eine sehr geringe Toxizität für den Menschen aufweist. Daher ist Genipin als Linker für eine Anwendung im Kontakt mit dem menschlichen Körper, insbesondere für eine Vernetzung von primären Aminogruppen als reaktive Seitengruppe aufweisenden Polymeren, wie z.B. Chitosan, besonders gut geeignet.

Die Vernetzung von Chitosan mit Genipin ist bereits bekannt. Ohne sich auf diese Theorie festlegen zu wollen, wird in er Literatur davon ausgegangen, dass die Vernetzung von Chitosan mit Genipin in zwei Bindungsreaktionen mit unterschiedlicher Geschwindigkeit erfolgt. Bei der schnelleren ersten Bindungsreaktion bewirkt eine primäre Aminogruppe eine Ringöffnung am C3-Atom des Genipins mit Bildung eines sekundären Amins am Stickstoff des Chitosanmoleküls sowie die Bildung einer Aldehydgruppe am benachbarten C1-Atom des Genipinmoleküls, welches danach zum sekundären Amin mit dem Stickstoffatom umlagert. Das Stickstoffatom des Chitosans befindet sich nun an jener Position, die vorher das Sauerstoffatom im Genipin innehatte. Die zweite langsamere, geschwindigkeitsbestimmende Bindungsreaktion ist eine nukleophile SN2-Substitution der Methylestergruppe des Genipins durch Bindung an ein weiteres Stickstoffatom. Diese Reaktion geht mit einer deutlichen Blaufärbung der Verbindung einher. Es hat sich überraschend gezeigt, dass das erfindungsgemäße, elektrostriktiv unterstützte Verfahren beide Bindungsreaktionen erheblich beschleunigt. Es wird davon ausgegangen, dass die Beschleunigung durch Bereitstellung von erhöhten Reaktionstemperaturen, Sauerstoffradikalen und $H^+$-Ionen erfolgt. Die Verwendung von Genipin als Linker in dem erfindungsgemäßen Verfahren hat zudem den Vorteil, dass im Zuge der Vernetzungsreaktion eine vermutlich durch Selbstpolymerisation des Genipins entstehende Blaufärbung des erzeugten physischen Objekts entsteht, welche die Unterscheidung des physischen Objekts von der Umgebung und damit den additiven Aufbau des physischen Objekts, die Detektion und die Quantifizierbarkeit mit optischen Mitteln erleichtert.

Es hat sich überraschend gezeigt, dass auch Geniposid, das Glycosid des Genipin, eine Vernetzungsreaktion mit Chitosan ergibt, wobei die Blaufärbung vermieden werden kann. Andere erfindungsgemäß geeignete Iridoide mit 8 bis 10 Kohlenstoffatomen sind beispielsweise Verbenalin aus den Früchten des Eisenkrauts oder Loganin aus der Gewöhnlichen Brechnuss (Strychnos nux-vomica). Selbstverständlich können auch synthetisch hergestellte Iridoide eingesetzt werden. Beispielsweise ist die synthetische Herstellung von Loganin Fachleuten bekannt. Weitere erfindungsgemäß geeignete, bioverträgliche Linker sind z.B. Harnstoff und Triamteren (6-Phenylpteridin-2,4,7-triamin).

In weiterer vorteilhafter Ausgestaltung des erfindungsgemäßen Verfahrens wird weiterhin wenigstens ein Zusatzpolymer in das elektrische Wechselstromfeld eingebracht. Die Zusatzpolymere haben den Zweck, dem zu bildenden physischen Objekt vor allem wählbare physikalische Eigenschaften hinsichtlich der Härte, Konsistenz, Leitfähigkeit für Wärme und Strom und Wasserbindungsfähigkeit oder auch medizinische Eigenschaften zu verleihen. Die Zusatzpolymere können insbesondere durch Pfropfcopolymerisation, Copolymersiation über reaktive Seitengruppen und/oder mittels zugefügter Linker oder durch Ionenbindung in dem physischen Objekt vernetzt werden.

In einer vorteilhaften Ausgestaltung der Erfindung sind Zusatzpolymere Polysaccharide, welche eine reduzierende terminale Aldehydgruppe aufweisen. Die reduzierende terminale Aldehydgruppe kann durch selektive Oxidation mit 0,1 N Iod in einer 0,1 N Kalilauge erhalten werden. Die Einbringung eines Zusatzpolymers mit einer reduzierenden terminalen Aldehydgruppe in das elektrische Wechselstromfeld führt zur Bildung von Pfropfcopolymeren. Das Zusatzpolymer ist bevorzugt ausgewählt aus der Gruppe bestehend aus Amylosen, Amylopektinen, Acemannanen, Dextranen, Arabinogalactanen, Galactanen, Galactoglucomannanen, Xanthanen, Carrageenen, Alginaten, Guargummi, Gummiarabikum, Glucosaminoglycane, Hyaluronsäure, Heparinen, Heparansulfaten, Chondroitinsulfat/Dermatansulfat, Keratansulfat, Agarose, Stärke und Gemischen daraus. Darüber hinaus können auch Polypeptide und Proteine, Nucleinsäuren und Ribonucleinsäure bzw. Verbindungen, die DNA- oder RNA-Bausteine enthalten, Immunoglobuline, Antigene, Epitope, molekulare Bindungsstellen von Proteinen und dergleichen als Zusatzpolymere eingesetzt werden.

In weiterer vorteilhafter Ausgestaltung des erfindungsgemäßen Verfahrens wird wenigstens eine Wirkstoffkomponente in das elektrische Wechselstromfeld eingebracht. Die Wirkstoffkomponente wird in Anhängigkeit von ihrer Form, Größe und Ladung in die sich bildenden Copolymer-Konglomerate eingeschlossen, wobei insbesondere Wirkstoffmoleküle, die Aminogruppen, Aldehydgruppen oder andere reaktive Gruppen aufweisen, auch kovalent in die erfindungsgemäßen Verbindungen eingebaut werden können.

Geeignete Wirkstoffkomponenten sind insbesondere pharmazeutisch wirksame Substanzen, die ausgewählt sind aus der Gruppe bestehend aus Abmagerungsmittel/Appetitzügler, Acidosetherapeutika, Aminosäuren (z. B. Histidin, Cystein) oder modifizierte Aminosäuren, Analeptika/Antihypoxämika, Analgetika/Antirheumatika, Anthelminthika, Antiallergika, Antianämika, Antiarrhythmika, Antibiotika/Antiinfektiva, Antidementiva (Nootropika), Antidiabetika, Antidota, Antiemetika/Antivertiginosa, Antiepileptika, Antihämorrhagika (Antifibrinolytika und andere Hämostatika), Antihypertonika, Antihypoglykämika, Antihypotonika, Antikoagulantia, Antimykotika, Antiparasitäre Mittel (intern), Antiphlogistika, Antitussiva/Expektorantia, Arteriosklerosemittel, Balneotherapeutika und Mittel zur Wärmetherapie, Betarezeptoren-, Calciumkanalblocker und Hemmstoffe des Renin-Angiotensin-Systems, Broncholytika/Antiasthmatika, Chemotherapeutika, Cholagoga und Gallenwegstherapeutika, Cholinergika, Corticoide (intern), Cyclodextrine und deren sulfatierte Derivate, Dermatika (intern), Diätetika/Ernährungstherapeutika, Diagnostika und Mittel zur Diagnosevorbereitung, Diuretika, durchblutungsfördernde Mittel, Elastin, Entwöhnungsmittel, Enzyminhibitoren, Enzympräparate und Transportproteine, Fibrinolytika, Gelatine, Geriatrika, Gichtmittel, Grippemittel, Gynäkologika, Hämorrhoidenmittel (Proktologika), Hepatika, Hypnotika/Sedativa, Hypophysen-, Hypothalamushormone, regulatorische Peptide und ihre Hemmstoffe, Immuntherapeutika und Zytokine, Infusions- und Standardinjektionslösungen, Organperfusionslösungen, Kardiaka, Karies-, Parodontosemittel und andere Dentalpräparate, Keratinoide, Kollagen, Koronarmittel, Laxantia, Lipidsenker, Neuraltherapeutika, Magen-Darm-Mittel, Migränemittel, Mineralstoffpräparate, Muskelrelaxantia, Narkosemittel, Nebenschilddrüsenhormone, Calciumstoffwechselregulatoren, Osteoporosemittel, Neuropathiepräparate und andere neurotrope Mittel, Neurotransmitter (z. B. Dopamin) oder modifizierte Neurotransmitter, Ophthalmika, Otologika, Parkinsonmittel und andere Mittel gegen extrapyramidale Störungen, Psychopharmaka, Sinusitismittel, Roborantia/Tonika, Schilddrüsentherapeutika, Sera, Immunglobuline und Impfstoffe, Sexualhormone und ihre Hemmstoffe, Silikon, Spasmolytika, Tenside, Thrombozytenaggregationshemmer, Tuberkulosemittel, Tricalciumphosphat, Hydroxylapatit, Umstimmungsmittel, Urologika, Venentherapeutika, Vitamine, Wundbehandlungsmittel, Zeolithe, Zytostatika und Metastasenhemmer.

Erfindungsgemäß werden das erste Polymer und das zweite Polymer sowie gegebenenfalls Linker, Zusatzpolymere und Wirkstoffkomponenten mithilfe einer Dosiervorrichtung in ein elektrisches Wechselstromfeld eingebracht, das zwischen wenigstens einer ersten Elektrode, die im Bereich der Auslassöffnung der Dosiervorrichtung angeordnet ist, und wenigstens einer zweiten Elektrode, die beabstandet von der ersten Elektrode angeordnet ist, erzeugt wird, wobei die Spannung, die Frequenz und die Stromstärke des elektrischen Wechselstromfeldes derart gewählt werden, dass die ersten reaktiven Seitengruppen des ersten Polymers und die zweiten reaktiven Seitengruppen des zweiten Polymers durch eine elektrostriktiv unterstützte chemische Reaktion miteinander verbunden werden.

Zur erleichterten Dosierung werden die ersten und zweiten Polymere, sowie weitere Komponenten, insbesondere Zusatzpolymere, Wirkstoffkomponenten oder Linker in einem fließfähigen Zustand dem elektrischen Wechselstromfeld zugeführt. Vorzugsweise werden die Komponenten in Lösung, gegebenenfalls in kolloidaler Lösung, als Dispersion, als Suspension, beispielsweise als Suspension von Fasern oder als Hydrogel vorgelegt und in das elektrische Wechselstromfeld eingebracht. Als Lösemittel bevorzugt sind Wasser, Alkohol und verdünnte wässrige Säuren, insbesondere Essigsäure, sowie Gemische daraus. Jedoch können auch kristallin vorliegende Polymere die erforderliche Fließfähigkeit aufweisen. Eine Einbringung von Feststoffen, insbesondere von Polymerfasern in das elektrische Wechselstromfeld ist ebenfalls möglich. In bestimmten Ausführungsformen kann es vorteilhaft sein, gasförmige Komponenten oder Komponenten in Form eines Aerosols, insbesondere ein Aerosol in einem Inertgas, dem elektrischen Wechselstromfeld zuzuführen.

Grundsätzlich kann das erfindungsgemäße Verfahren mit Frequenzen bis zu 100 GHz betrieben werden. Vorteilhafterweise wird ein elektrisches Wechselstromfeld mit einer Frequenz von 200 kHz bis 1 GHz angelegt, wobei die Verwendung sehr hoher Frequenzen auf den Einsatz außerhalb des menschlichen oder tierischen Körpers begrenzt sind. Für die Anwendung am menschlichen oder tierischen Körper sind Frequenzen von 200 kHz bis 350 kHz ganz besonders bevorzugt. Die im Einzelfall geeignete Frequenz hängt nicht nur von der Temperatur- und Strahlungsempfindlichkeit des vorgesehenen Bildungsortes sondern auch von der relativen Permittivität der eingesetzten ersten und zweiten Polymere ab. Die relative Permittivität der Polymere sowie weitere dielektrische Eigenschaften der dem elektrischen Wechselstromfeld zuzuführenden Komponenten werden vorteilhafterweise vorab mit dem Fachmann geläufigen Mitteln ermittelt. Bei Anwendungen am menschlichen und tierischen Körper ist es außerdem erforderlich, vorab die dielektrischen Eigenschaften des Bildungsorts zu bestimmen, um Schädigungen, insbesondere eine Carbonisierung des Gewebes an dem vorgesehenen Bildungsort vermeiden zu können.

Das erfindungsgemäße Verfahren wird typischerweise mit einer Leistung von 40 bis 800 Watt, vorzugsweise 40 bis 200 Watt betrieben.

Grundsätzlich müssen die eingesetzten Frequenzen und Energien ausreichend hoch gewählt werden, dass eine Vernetzung der in das elektrische Wechselstromfeld eingebrachten Komponenten miteinander und eine Vernetzung des gebildeten physischen Objekts mit dem Bildungsort, z.B. dem menschlichen oder tierischen Gewebe oder einem bereits gefügten physischen Objekt, gewährleistet ist.

Bei der Untersuchung der Reaktion in Wechselstromfeldern mit unterschiedlichen Frequenzen und Feldenergiestärken konnte eine deutliche Beschleunigung der Vernetzungsreaktion bei Erhöhung der Energie im elektrischen Feld beobachtet werden. Proportional mit der Energie des angelegten elektrischen Feldes stieg auch die Wärmeentwicklung in dem Polymergemisch und damit der dielektrische Verlust in dem System.

Es konnte beobachtet werden, dass die Polymere bei einer Annäherung an das Wechselstromfeld durch die von dem Wechselstromfeld erzeugte elektrostriktive Kraft in das Wechselstromfeld hineingezogen werden. Es kommt dabei zu einer sichtbaren Mischung der ersten und zweiten Polymere.

Ohne sich auf diese Theorie festlegen zu wollen, wird davon ausgegangen, dass die Polymere in dem Wechselstromfeld eine inverse piezoelektrische Verformung erfahren und entsprechend oszillieren und dass die Ausbildung kovalenter Bindungen in dem Wechselstromfeld durch den dielektrischen Verlust innerhalb der Polymere thermisch begünstigt wird. Bei bestimmten Polymeren kann eine weitere Begünstigung der Bildung kovalenter Bindungen gegebenenfalls zudem durch entstehende Radikale oder die Entstehung energiereicher Plasmen erfolgen.

Um unerwünschte Nebenreaktionen in dem elektrischen Wechselstromfeld zu minimieren, kann es vorteilhaft sein, die Vernetzungsreaktion unter Schutzgasatmosphäre durchzuführen. Geeignete Schutzgase sind beispielsweise Kohlendioxid ($CO_2$) und Stickstoff ($N_2$).

Auch eine Bedampfung des in dem Verfahren zu bildenden physischen Objekts mit einem plasmabildenden Gas während des Fügevorgangs kann vorteilhaft sein. Geeignete Gase, die in einem hochfrequenten Wechselstromfeld ein Plasma bilden, sind beispielsweise Stickstoff, Wasserstoff, Helium, Neon, Argon, Krypton, Xenon, Radon. Das Plasma bewirkt insbesondere eine Erwärmung der Oberfläche des bereits gefügten Bildungsortes und fördert somit die Vernetzung des bereits gefügten Bildungsortes mit einem in einem anschließenden Fertigungsschritt neu entstehenden physischen Objekt.

In einer bevorzugten Ausführungsform der Erfindung wird die Veränderung der dielektrischen Eigenschaften in dem angelegten elektrischen Feld möglichst kontinuierlich gemessen. Die dielektrischen Eigenschaften umfassen insbesondere den Dielektrizitätskoeffizienten, den dielektrischen Verlustfaktor und den dielektrischen Durchschlag. Die dielektrischen Eigenschaften sind charakteristisch für die Komponenten der Vernetzungsreaktion, insbesondere die eingesetzten ersten und zweiten Polymere. Damit wird der Fortschritt der kovalenten Vernetzung des ersten Polymers mit dem zweiten Polymer erfasst, wobei die Polarisierbarkeit der ersten und zweiten Polymere in dem Maß abnimmt, in dem die Vernetzung der beiden Polymere zunimmt. Somit lassen sich insbesondere physikalische Eigenschaften, z.B. die Elastizität, des zu fügenden physischen Objekts kontrollieren. Durch die Messung der dielektrischen Eigenschaften in dem angelegten elektrischen Feld können auch die dielektrischen Eigenschaften des Bildungsortes erfasst werden. Dies ist bei einer Anwendung bei tierischem und menschlichem Gewebe als Bildungsort besonders wichtig, um Schäden des Gewebes bei der Vernetzung aufgrund eines dielektrischen Durchschlags, aber auch um eine unvollständige Vernetzung des Bildungsortes mit dem gebildeten physischen Objekt zu vermeiden.

Die erfindungsgemäße Vorrichtung zur additiven Fertigung und Applikation eines räumlich ausgebildeten physischen Objekts an einem vorbestimmbaren Bildungsort weist eine räumlich verlagerbare Dosiervorrichtung auf, die einen Mündungsraum mit einer Auslassöffnung und wenigstens einen Zuführkanäle zur dosierbaren Zuführung von Komponenten in den Mündungsraum aufweist, sowie eine Wechselstromgeneratoreneinheit, an die wenigstens eine erste Elektrode, die im Bereich des Mündungsraums angeordnet ist, und wenigstens eine zweite Elektrode, die beabstandet von der ersten Elektrode angeordnet ist, angeschlossen ist, wobei die Spannung, die Frequenz und die Stromstärke jedes von der Wechselstromgeneratoreneinheit erzeugten elektrischen Wechselstromfeldes wählbar sind.

Mit der erfindungsgemäßen Vorrichtung können in den Mündungsraum eingebrachte Komponenten, insbesondere Polymere, Linker, Zusatzpolymere und Wirkstoffkomponenten, durch Anlegen eines elektrischen Wechselstromfeldes unterstützt von elektrostriktiven Wirkungen vernetzt werden.

In vorteilhafter Ausgestaltung der Erfindung sind wenigstens zwei Zuführkanäle zur getrennt dosierbaren Zuführung einer ersten fließfähigen Komponente und einer zweiten fließfähigen Komponente in den Mündungsraum vorgesehen. Damit werden unerwünschte Nebenreaktionen der Komponenten, die beim Mischen der Komponenten außerhalb des elektrischen Wechselstromfeldes auftreten könnten, vermieden.

Die zweite Elektrode ist vorteilhafterweise ebenso wie die erste Elektrode im Bereich des Mündungsraums angeordnet, besonders bevorzugt sind beide Elektroden einander gegenüberliegend im Bereich der Auslassöffnung des Mündungsraumes angeordnet, sodass beim Anlegen des Wechselstroms ein bipolares Wechselstromfeld entsteht. Diese Elektrodenanordnung, bei der beide Gegenelektroden eines Elektrodenpaars beabstandet voneinander im Bereich des Mündungsraums der Dosiervorrichtung angeordnet sind, wird im Rahmen der Erfindung als bipolar bezeichnet.

Alternativ oder zusätzlich zu einer weiteren ersten Elektrode kann die zweite Elektrode im Wesentlichen unabhängig von der Dosiervorrichtung räumlich verlagerbar sein, sodass ein monopolares Wechselstromfeld entsteht, wenn eine Wechselspannung angelegt wird. Diese Elektrodenanordnung, bei der eine erste Elektrode eines Elektrodenpaars im Bereich des Mündungsraums der Dosiervorrichtung angeordnet, und deren Gegenelektrode im Bereich des physischen Objekts angeschlossen ist, wird im Rahmen der Erfindung als monopolar bezeichnet.

In weiterer vorteilhafter Ausgestaltung der Erfindung sind mehrere erste und zweite Elektroden vorgesehen, sodass mehrere bipolare und monopolare Wechselstromfelder im Wechsel und/oder gleichzeitig betrieben werden können. Damit kann die Ausbildung von Feldüberlagerungen in der Art von Lissajous-Figuren ermöglicht werden, welche Einfluss auf die räumliche Ausbildung des bei der Vernetzung entstehenden physischen Körpers haben.

In weiterer vorteilhafter Ausgestaltung der Erfindung weist die Vorrichtung weiterhin eine Extrudervorrichtung auf, die innerhalb eines Führungskanals verschieblich angeordnet ist und in den Mündungsraum ein- und ausfahrbar ist, sodass in dem Mündungsraum vorliegende fließfähige Komponenten in Richtung der Auslassöffnung verlagerbar sind.

In noch weiterer vorteilhafter Ausgestaltung der Erfindung kann die Extrudervorrichtung im Bereich ihres in den Mündungsraum weisenden Endes eine weitere erste Elektrode aufweisen. Um ein monopolares Wechselstromfeld zu erzeugen, ist die auf dem Extruder angeordnete weitere erste Elektrode an einen Wechselstromgenerator angeschlossen, wobei die zugehörige zweite Elektrode eine Neutralelektrode ist, welche im Wesentlichen unabhängig von der Dosiervorrichtung verlagerbar ist und im Bereich des vorgesehenen Bildungsortes oder an dem bereits additiv gefügten physischen Objekt angebracht ist. Zur Erzeugung eines bipolaren Wechselstromfeldes kann die auf dem Extruder angeordnete weitere erste Elektrode mit einer im Bereich des Mündungsraums der Dosiervorrichtung angeordneten Elektrode als zweite Elektrode an einen Wechselstromgenerator gekoppelt sein.

Für die Erzeugung bipolarer Wechselstromfelder ist es bevorzugt, dass die erfindungsgemäße Vorrichtung wenigstens ein Elektrodenpaar aufweist, das im Bereich der Auslassöffnung des Mündungsraums der Dosiervorrichtung angeordnet ist. Die Position, Lage und Ausrichtung des elektrischen Wechselstromfeldes sowie die gezielte Überlagerung mehrerer elektrischer Wechselstromfelder kann die Ausbildung bestimmter räumlicher Strukturen in dem gefügten physischen Objekt begünstigen.

In weiterer vorteilhafter Ausgestaltung weist die erfindungsgemäße Vorrichtung eine Messeinheit zur Bestimmung und Überwachung der dielektrischen Eigenschaften der Komponenten in dem elektrischen Wechselstromfeld auf. Vorteilhafterweise werden für die Bestimmung und Überwachung der dielektrischen Eigenschaften eine erste Elektrode und eine zweite Elektrode genutzt, die im Bereich des Mündungsraumes der Dosiervorrichtung angeordnet sind. Werden die im Bereich des Mündungsraumes angeordneten Messelektroden nahe genug an den vorgesehenen Bildungsort herangeführt, so können mit der Messeinheit auch die dielektrischen Eigenschaften des Bildungsortes vorab bestimmt werden. Dies ist insbesondere dann von Vorteil, wenn der Bildungsort einen menschlichen oder tierischen Körperteil oder ein Körpergewebe umfasst, das bei Anwendung zu hoher Frequenzen und Energien in dem erfindungsgemäßen Verfahren Schaden nehmen könnte. Für die Anwendung als chirurgisches Arbeitsgerät ist es erforderlich, die Messfunktionen zur Bestimmung der dielektrischen Eigenschaften der Komponenten und des Bildungsortes am menschlichen oder tierischen Körper engmaschig zu erfassen und mit den erfassten Werten insbesondere die Leistung, Frequenz, Pumpen, Polymerzusammensetzung, Elektrodenkonfiguration, Verhältnis und Überlagerung verschiedener Frequenzen zu steuern, um unzureichende Vernetzungen mit funktionellen Gruppen des Körpergewebes selbst oder dielektrischer Durchschlag mit Zerstörung von Körpergewebe, additiv gefertigtem physischem Objekt und deren Funktionen zu vermeiden.

Die erfindungsgemäße Vorrichtung ist in einer ganz besonders bevorzugten Ausführungsform als endoskopisches Arbeitsgerät ausgebildet. Mit dem endoskopischen Arbeitsgerät kann eine Anwendung des erfindungsgemäßen Verfahrens vorteilhaft am menschlichen oder tierischem Körper erfolgen. Dabei ist der Mündungsraum, an dem das elektrische Wechselstromfeld angelegt ist und dem die Komponenten zugeführt werden, im Bereich der Spitze des endoskopischen Arbeitsgeräts angeordnet. Es versteht sich, dass ein derartiges endoskopisches Arbeitsgerät zum Schutz des zu behandelnden Patienten bestimmte Bedingungen erfüllen muss. Insbesondere sollte die Vorrichtung gasdicht und sterilisierbar ausgebildet sein, einen Zuführkanal für Schutzgas aufweisen und vorzugsweise eine Messeinheit zur Bestimmung und Überwachung dielektrischer Eigenschaften vom Komponenten in dem elektrischen Wechselstromfeld aufweisen, um körperverträgliche Frequenzen einstellen zu können, sodass Gewebeschädigungen durch Verkohlung vermieden werden können.

Die Erfindung wird nachfolgend beispielhaft anhand von Figuren näher erläutert. Es zeigen:

Figur 1 eine erfindungsgemäße Vorrichtung in Schnittansicht entlang einer vertikalen Schnittebene in schematischer Darstellung;

Figuren 1A bis 1C horizontale Schnittansichten des mündungsnahen Bereichs der Vorrichtung gemäß Fig. 1 und Fig. 2 entlang den Linien A-A', B-B' und C-C';

Figur 2 die erfindungsgemäße Vorrichtung aus Fig. 1 in Schnittansicht entlang einer vertikalen Schnittebene, die gegenüber der Schnittebene aus Fig. 1 um 90° gedreht ist, in schematischer Darstellung;

Figuren 3A bis 3C die erfindungsgemäße Vorrichtung aus Fig. 1 in Schnittansicht entlang einer vertikalen Schnittebene in schematischer Darstellung mit unterschiedlichen Extruderpositionen; und

Figur 4 eine erfindungsgemäße Vorrichtung in vertikaler Schnittansicht zusammen mit einer schematischen Darstellung einer für die Messung der Dielektrizitätskonstanten geeigneten Wechselspannungsmessbrücke.

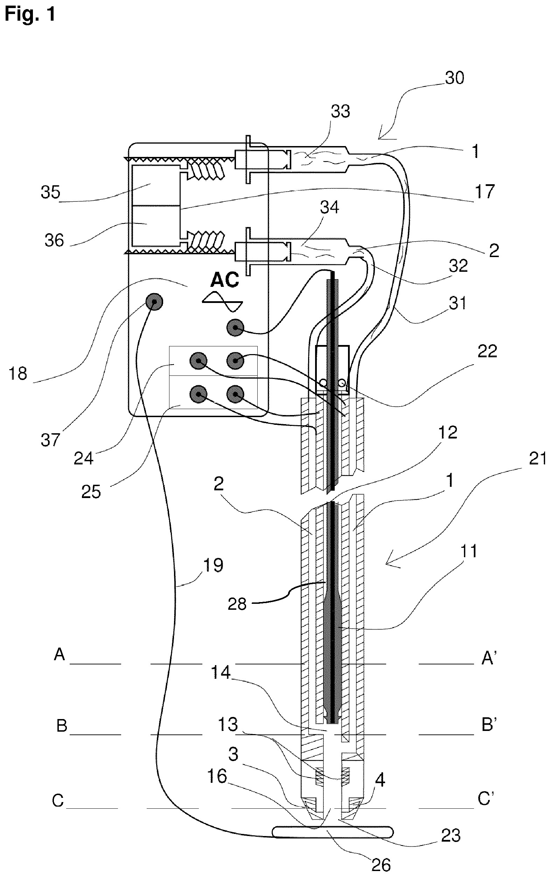

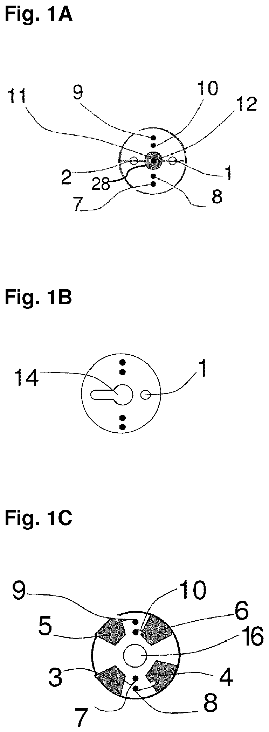

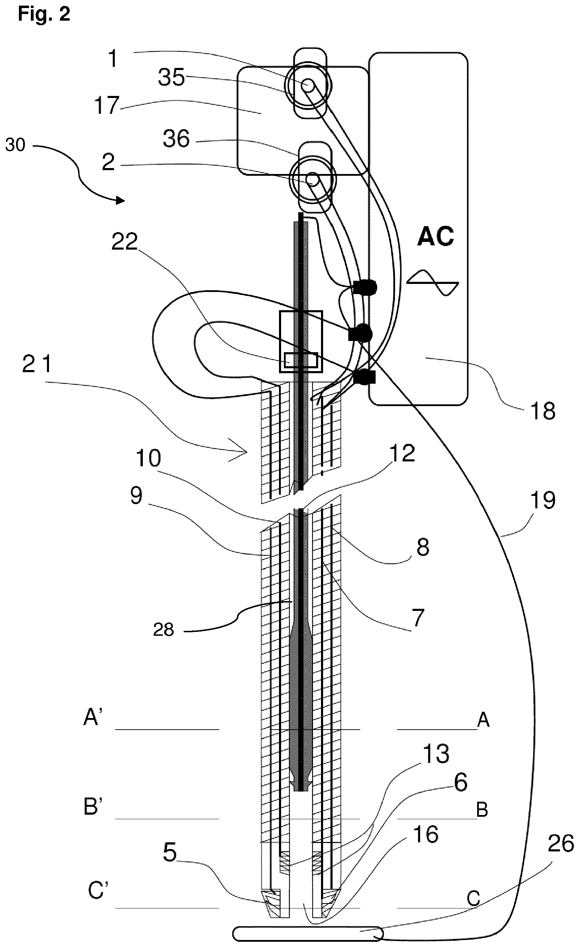

Fig. 1 und Fig. 2 zeigen eine Ausführungsform der erfindungsgemäßen Vorrichtung 30 mit einer Dosiervorrichtung, die eine Pipettiervorrichtung 21 und eine Dosier- und Pumpeinheit 17 aufweist, und einer Wechselstromgeneratoreneinheit 18 in zwei um 90° zueinander gedrehten vertikalen Schnittansichten. In den Fig. 1A bis 1C sind horizontale Schnittansichten des mündungsnahen Bereichs der Pipettiervorrichtung 21 entlang der in den Figuren 1 und 2 dargestellten Linien A-A', B-B' und C-C' dargestellt. Fig. 1 und 2 zeigen im oberen Teil eine Dosier- und Pumpeinheit 17 für die Injektion einer ersten fließfähigen Komponente 31 und einer zweiten fließfähigen Komponente 32 in einen ersten Zuführkanal 1 bzw. in einen zweiten Zuführkanal 2. Die ersten und zweiten Komponenten umfassen in der Regel die ersten und zweiten Polymerlösungen 31, 32, können aber auch eine Polymerlösung als erste Komponente und eine Linkerlösung als zweite Komponente umfassen. Selbstverständlich können noch weitere Zuführkanäle vorgesehen sein, um gegebenenfalls weitere Komponenten, wie z.B. Linker, Wirkstoffkomponenten oder Zusatzpolymere getrennt von den ersten und zweiten Komponenten 31, 32 zu transportieren oder weitere Reagenzien für anschließende Verfahrensschritte, z.B. Reduktionsmittel zur Reduktion von Iminbindungen bereitzustellen. Es ist aber auch möglich, weitere Komponenten einer der Polymerlösungen zuzumischen und gemeinsam mit dieser zu transportieren.

Die Dosier- und Pumpeinheit 17 weist erste und zweite Injektionsspritzen 33, 34 auf, welche durch Motoren 35, 36 betätigbar sind. Die ersten und zweiten Polymerlösungen 31, 32 werden in den Injektionsspritzen 33, 34 vorgelegt und von den Motoren 35, 36 durch die Zuführkanäle 1, 2 in einen gemeinsamen Mündungsraum 16 im Bereich der Spitze der Pipettiervorrichtung 21 gepumpt.

In der Pipettiervorrichtung 21 ist ein in den Mündungsraum 16 der Pipettiervorrichtung 21 mündender Führungskanal 28 vorgesehen, in den eine Extrudervorrichtung 11 verschieblich eingeführt ist. Wie auch in Fig. 1A erkennbar ist, schließt die Extrudervorrichtung den Führungskanal 28 zum den Mündungsraum 16 hin im Wesentlichen dicht. Die Verschiebung der Extrudervorrichtung 11 durch den Führungskanal 28 in den Mündungsraum 16 hinein bis zur Auslassöffnung 23 und wieder hinaus ist mittels eines dritten Motors 22 möglich. Durch die Verlagerung der Extrudervorrichtung 11 nach unten in Richtung einer Auslassöffnung 23 des Mündungsraums 16 werden in den Mündungsraum 16 eingebrachte Komponenten zur Auslassöffnung 23 hin verlagert.

Wahlweise kann die Extrudervorrichtung auch vollständig aus dem Führungskanal 28 entnommen werden und der Führungskanal 28 zum Einführen eines festen oder filamentösen ersten oder zweiten Polymers, z.B. Viskosefasern oder Cellulosefasern, in den Mündungsraum 16 genutzt werden.

Die Motoren 22, 35, 36, die Wechselstromgeneratoreneinheit 18 sowie gegebenenfalls weitere, hier nicht näher dargestellte Mittel zur Positionierung der erfindungsgemäßen Vorrichtung in Bezug zu einem vorbestimmten Bildungsort 26 für das zu erzeugende physische Objekt werden von einer ebenfalls nicht dargestellten Recheneinheit gesteuert.

Im Bereich der Auslassöffnung 23 des Mündungsraums 16 der Pipettiervorrichtung 21 sind vier Wechselstromelektroden 3, 4, 5 und 6 angebracht. Wie aus Fig. 1C ersichtlich ist, sind die erste Elektrode 3 und die zweite Elektrode 6 bezüglich des Mündungsraums 16 in einer gegenüberliegenden Position angeordnet. Die danebenliegende erste Elektrode 4 und zweite Elektrode 5 nehmen bezüglich des Mündungsraums 16 ebenfalls eine einander gegenüberliegende Position ein. Somit können in dieser Anordnung zwei sich überlagernde bipolare Wechselstromfelder gebildet werden.

Im Endbereich der Auslassöffnung 23 des Mündungsraumes 16 sind die Elektrodenpaare 3,6 und 4,5 dergestalt nebeneinander angebracht, dass sich bei angelegtem Wechselstrom deren jeweilige elektrischen Feldlinien im Mündungsraum 16 unter einem bekannten Winkel überkreuzen. Dieser Winkel beträgt in den Fig. 1 und 2 unter Annahme zweier homogener elektrischer Wechselstromfelder angenähert 90°.

Die erste Elektrode 3 ist über eine Leitung 8 und deren zugehörige zweite Elektrode 6 über eine Leitung 10 mit einem ersten Wechselstromgenerator 24 der Wechselstromgeneratoreneinheit 18 elektrisch leitend verbunden. Die erste Elektrode 4 ist über eine Leitung 7 und deren zugehörige zweite Elektrode 5 über eine Leitung 9 an den einen zweiten Wechselstromgenerator 25 der Wechselstromgeneratoreneinheit 18 angeschlossen. Wie aus Fig. 1A und Fig. 1B ersichtlich ist, sind die Leitungen 7, 8, 9, 10 ebenso wie die Zuführkanäle 1, 2 innerhalb der Pipettiervorrichtung 21 geführt.

In einem inneren Bereich des Mündungsraums 16 ist ein weiteres Elektrodenpaar 13 vorgesehen, das in dem in Fig. 1 und 2 dargestellten Ausführungsbeispiel nicht genutzt wird.

Das Elektrodenpaar 13 eignet sich aufgrund seiner Lage im Innern des Mündungsraums 16 ohne direkten Kontakt zur Umgebung für die Anwendung besonders energiereicher elektrischer Wechselstromfelder mit hohen Frequenzen.

Eine weitere erste Elektrode 12 ist im Bereich des in den Mündungsraum 16 weisenden Endes der Extrudervorrichtung 11 angeordnet. Diese erste Elektrode 12 kann in Kombination mit einer zweiten Elektrode, die als Neutralelektrode 19 bezeichnet wird und die im Wesentlichen unabhängig von der Pipettiervorrichtung räumlich verlagerbar ist, zum Aufbau eines monopolaren Wechselstromfeldes genutzt werden. In Fig. 1 und 2 ist die Neutralelektrode 19 im Bereich des für das zu fügende physikalische Objekt vorgesehenen Bildungsortes 26 angeschlossen.

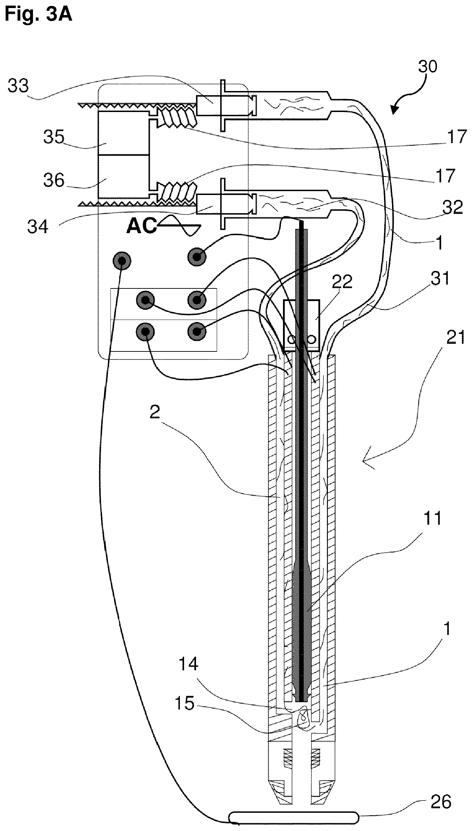

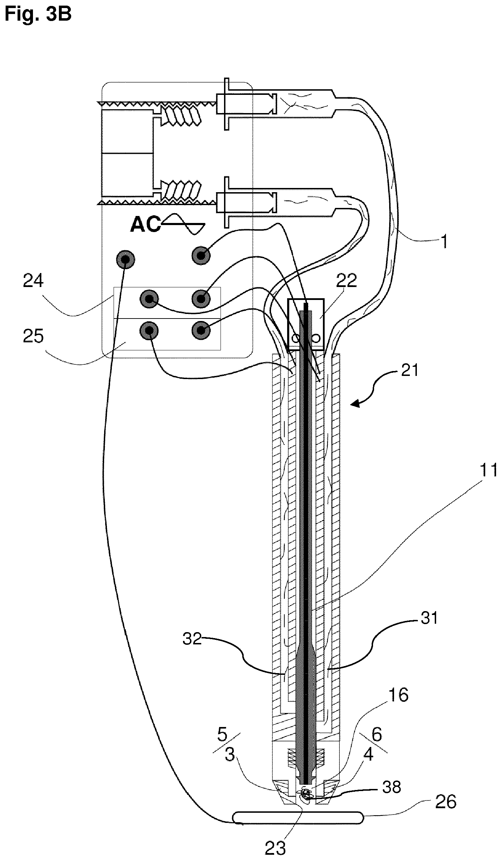

Die Fig. 3A bis 3C zeigen drei fortlaufende Schritte eines elektrostriktiv unterstützten Fertigungsvorgangs an einer erfindungsgemäßen Vorrichtung 30. Fig. 3A zeigt die Extrudervorrichtung 11 in vollständig aus dem Mündungsraum 16 herausgefahrener Position, sodass eine erste Mündung 15 des ersten Zuführkanals 1 und eine zweite Mündung 14 des zweiten Zuführkanals 2 vollständig geöffnet sind. Die ersten und zweiten Injektionsspritzen 33, 34 sind mit der ersten bzw. zweiten fließfähigen Polymerkomponente 31, 32 und gegebenenfalls weiteren Komponenten gefüllt, sodass die ersten und zweiten Motoren 35, 36 die fließfähigen Polymerkomponenten 31, 32 in einer vorbestimmbaren Menge durch die jeweiligen Zuführkanäle 1, 2 in den Mündungsraum 16 der Pipettiervorrichtung 21 pumpen können. Fig. 3B zeigt, dass das in den Mündungsraum 16 eingebrachte Gemisch 38 der Polymerkomponenten 31, 32 durch Einfahren der Extrudervorrichtung 11 in den Mündungsraum 16 in Richtung der Auslassöffnung 23 der Pipettiervorrichtung 21 transportiert wird, an der auch die Elektrodenpaare 4, 5 und 3,6 angeordnet sind. Der erste Wechselstromgenerator 24 erzeugt danach zwischen den Elektroden 3 und 6 ein elektrisches Wechselstromfeld mit einer wählbaren ersten Frequenz f1. Der zweite Wechselstromgenerator 25 erzeugt zwischen den Elektroden 4 und 5 ein Wechselstromfeld mit der zweiten Frequenz f2, wobei das Verhältnis zwischen f1 und f2 einen Bruch aus ganzen Zahlen ergibt.

Die elektrostriktiv wirkende Kraft der elektrischen bipolaren und monopolaren Wechselstromfelder zieht die ersten und zweiten Polymere aus den zugehörigen Zuführkanälen in das elektrische Wechselstromfeld hinein und sorgt für eine sichtbare Mischung und Vernetzung der Komponenten. Es versteht sich deshalb, dass die Vorrichtung 30 grundsätzlich auch ohne die Verwendung einer Extrudervorrichtung 11 eingesetzt werden kann. Es ist daher grundsätzlich möglich, die erfindungsgemäße Vorrichtung in der Art eines Druckkopfes mit einer Vielzahl von Druckpositionen, z.B. mit bis zu 50 Druckpositionen, typischerweise mit 4 bis 12 Druckpositionen zu betreiben, wobei jeder Druckposition ein bipolares elektrisches Wechselstromfeld zugeordnet ist.

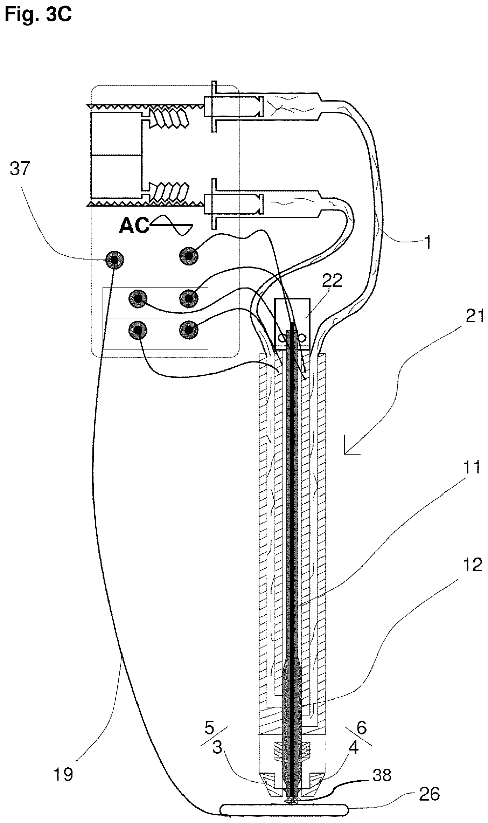

Fig. 3C zeigt den abschließenden Schritt des Fertigungsvorgangs. Dabei wird die erste Elektrode 12, die an der Extrudervorrichtung 11 angeordnet ist, an einen Wechselstromgenerator 37 angeschlossen, dessen Gegenelektrode eine oder mehrere Elektroden ausgesucht aus den Elektroden 3,4,5,6 oder die mit dem bereits additiv gefügten Körper verbundene Neutralelektrode 19 ist. Gleichzeitig wird das Polymergemisch 38 mittels der Extrudervorrichtung 11 nach unten auf den Bildungsort 26 gedrückt. Danach wird die erste Elektrode 12 vom Wechselstromgenerator getrennt und die Extrudervorrichtung 11 in die in Fig. 3A dargestellte Ausgangsposition zurückgefahren. Anschließend kann der nächste additive Fertigungsvorgang erfolgen, um das zu fügende physische Objekt aufzubauen.

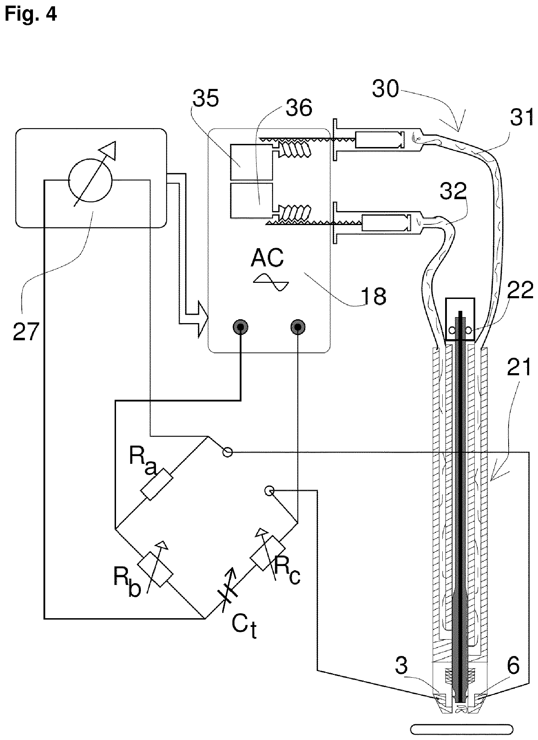

Fig. 4 zeigt eine erfindungsgemäße Vorrichtung 30 mit einer geeigneten Messbrückenschaltung zur Ermittlung und zur Überwachung der dielektrischen Eigenschaften des in dem elektrischen Wechselstromfeld befindlichen Dielektrikums. Bei der Messbrückenschaltung handelt es sich um eine Wechselspannungsbrücke mit Widerständen Ra, Rb, Rc, einem Kondensator Ct und einem Messverstärker 20. Dabei sind die Widerstände Rb und Rc sowie die Kapazität Ct einstellbar. Die Messbrücke ist an die erste Elektrode 3 und die zweite Elektrode 6 angeschlossen, welche im Bereich der Auslassöffnung 23 des Mündungsraums 16 der Pipettiervorrichtung 21 angeordnet sind. In dem Mündungsraum 16 befindet sich das Polymergemisch 29, dessen dielektrische Eigenschaften bestimmt und/oder überwacht werden sollen.

Die Wechselspannungsmessbrücke erlaubt es, eine gesuchte Kapazität Cx und einen dielektrischen Verlustfaktor tan δ zu bestimmen. Wenn die Wechselspannungsbrücke abgeglichen ist, so ergibt sich

$$Cx \quad = \quad Ct \; * \; Rb/Ra$$

und

$$\tan \delta \quad = \quad \omega \; * \; Ct \; * \; Rc$$

Bei der erfindungsgemäßen Vernetzung der ersten und zweiten Polymere 31, 32 werden Rb, Ct und Rc solchermaßen eingestellt, dass die Wechselspannungsbrücke abgeglichen ist, sodass Cx und tan δ ermittelt werden können. Dabei können die Kapazität Ct und der Widerstand Rc vorab an die von dem Wechselstromgenerator 18 vorgegebenen Frequenzen angepasst werden. Der Widerstand Rb kann auf einen gewünschten Widerstandswert eingestellt werden, bei dem die Vernetzungsreaktion in dem Mündungsraum 16 der Pipettiervorrichtung 21 beendet werden soll. Wenn die Wechselspannungsbrücke abgeglichen ist, so wird dies durch den Messverstärker 27 erfasst und Cx und tan δ berechnet. Anschließend wird der nächste Fügevorgang initialisiert. Dem Fachmann sind digital arbeitende Messbrücken bekannt, in denen Rb, Rc und Ct einstellbar sind und die einer Steuereinheit die Kapazität Cx und den dielektrischen Verlustfaktor tan δ digital übermitteln können.

Die Erfindung wird im Folgenenden weiter anhand von Beispielen erläutert.

Beispiele:

Beispiel 1

5 g Carboxymethylcellulose Natriumsalz werden in 20 mL $NaIO_4$ 0,5 mol/L gelöst und es werden 0,02 mL 1-Propanol zugemischt. Das Gefäß wird mit Aluminiumfolie lichtdicht verschlossen und 72 Std. bei 30 °C geschüttelt. Es entsteht ein klares, Carboxymethylcellulosedialdehyd enthaltendes Gel, welches dreimal mit 100 mL destilliertem Wasser filtriert wird.

2 g Chitosan werden in 50 mL 0,2N Essigsäure gelöst.

Auf eine mit Influenzgenerator negativ geladene Glasplatte werden 5 mL der Chitosanlösung aufgetropft und über 72 Std. bei 30 °C stehen gelassen. An der Spitze einer drei Injektionskanäle führenden Pipettiervorrichtung (EVICEL, Ethicon, Johnson & Johnson Medical, Ltd.) wird eine ringförmige Stahlelektrode befestigt, welche an einem Ausgang eines Wechselstromgenerators angeschlossen wird. Der andere Ausgang des Generators wird an die mit Chitosan beschichtete Glasplatte angeschlossen. Der Wechselstromgenerator wird mit einer Frequenz von 350 kHz und einer Leistung von 40 W betrieben. Pipettiervorrichtung und Elektrode werden bis auf 5 mm an die Glasplatte herangefahren. 50 μL der jeweiligen Polymerkomponenten werden durch separate Zuführkanäle von oben in Richtung der Glasplatte pipettiert. Unter Dampfaustritt wächst auf der Glasplatte ein Tropfen, der sich zunehmend dunkler färbt und aushärtet. Die angelegte Spannung wird unterbrochen und 20 μL einer 5%igen Ascorbinsäurelösung durch den dritten Injektionskanal aufgebracht. Der Vorgang wird direkt neben dem vorher gefügten Tropfen 25 Mal wiederholt. Danach wird das entstandene physische Objekt mit Wasser und 10%iger Ethanollösung mehrfach gespült. Bei geringer Flüssigkeitsaufnahme bleibt seine Form stabil.

Beispiel 2:

5 g Carboxymethylcellulose Natriumsalz werden in 20 mL $NaIO_4$ 0,5 mol/L gelöst und 0,02 mL 1-Propanol zugemischt. Das Gefäß wird mit Aluminiumfolie lichtdicht verschlossen, 72 Std. bei 30 °C geschüttelt und das entstandene Carboxymethylcellulosedialdehyd enthaltende Gel wird wie in Bespiel 1 beschrieben dreimal filtriert.

2 g Chitosan werden in 50 mL 0,2 N Essigsäure gelöst.

Jeweils 10 mL der beiden Komponenten werden auf eine Glasplatte pipettiert, gemischt und 15 min bei 25 °C Raumtemperatur stehen gelassen. Zwei Stahlplättchen (1 cm x 0,5 cm) werden als Elektroden an den Wechselstromgenerator angeschlossen, der mit einer Frequenz von 350 kHz und einer Leistung von 40 Watt betrieben wird. Bei einer Annäherung der Elektroden an den Tropfen bis auf 5 mm wird der Tropfen in das elektrische Feld hineingezogen. Der Tropfen verfärbt sich unter Dampfaustritt. Das entstandene physische Objekt bleibt für Wasser und eine 10%ige Ethanollösung bei geringer Flüssigkeitsaufnahme formstabil.

Beispiel 3

Der Versuch wird mit den Komponenten wie in Beispiel 2 bei einer Frequenz von 350 kHz und einer Leistung von 150 Watt durchgeführt. Bei Annäherung der Elektroden wird der Tropfen in das bestehende elektrische Feld gezogen, verfärbt sich sofort dunkelbraun und härtet aus. Das entstandene physische Objekt ist hart, spröde und bricht bei Druckbelastungen.

Beispiel 4

10 g Carboxymethylcellulose Natriumsalz werden in 40 mL $NaIO_4$ 0,5 mol/L gelöst und es werden 0,04 mL 1-Propanol zugemischt. Das Gefäß wird mit Aluminiumfolie lichtdicht verschlossen und 72 Std. bei 30 °C geschüttelt. Das entstandene, klare Carboxymethylcellulosedialdehyd enthaltende Gel wird dreimal mit 100 mL Wasser filtriert. In das entstandene werden 2 g Carboxymethylcellulose gegeben und bei Raumtemperatur über Nacht geschüttelt.

2 g Chitosan werden in 50 mL 0,2 N Essigsäure gelöst.

Die Pipettiervorrichtung aus Beispiel 1 wird an den Wechselstromgenerator mit 350 kHz und 100 Watt angeschossen, dessen anderer Ausgang mit einer wie in Beispiel 1 beschrieben mit Chitosan beschichteten Glasplatte in Verbindung steht. Je 50 μL der beiden Polymergemische werden aus 5 mm Höhe durch separate Kanäle in Richtung der Glasplatte pipettiert. Unter Dampfbildung wächst auf der Glasplatte ein bernsteinfarbener Tropfen. Nach Abschalten der Stromzufuhr werden 20 μL einer 5%igen Ascorbinsäurelösung aufgebracht. Der Vorgang wird wie in Beispiel 1 mehrfach wiederholt.

Das entstandene physische Objekt wird dreimal mit Wasser und mit einer 10%igen Ethanollösung gespült. Das gebildete Objekt zeigt eine größere Wasseraufnahme als das Objekt aus Beispiel 1, bleibt jedoch in seiner Form stabil.

Beispiel 5

2 g Chitosan werden in 50 mL 0,2 N Essigsäurelösung gelöst und 24 Stunden bei 25° C stehen gelassen bis keine Luftbläschen mehr sichtbar sind. Das Chitosangel wird in den zweiten Zuführkanal der Pipettiervorrichtung gefüllt. Der Versuchsraum wird abgedunkelt.

10 mg Genipin werden in 4 ml $H_2O$ gelöst und sofort in die mit dem ersten Zuführkanal verbundene Pipettiervorrichtung gefüllt.

100 μl der Chitosanlösung und 5 μl der Genipinlösung werden in den Mündungsraum der Pipettiervorrichtung zwischen zwei Wechselstromelektroden eingebracht. An die Elektroden wird ein Wechselstrom mit einer Frequenz von 340 kHz mit 40 W angelegt und die Pipettenspitze wird an eine Bodenplatte angenähert. Die Leistung des Wechselstromgenerators wird schrittweise unter Dampfaustritt erhöht bis in dem Gemisch vereinzelte Lichtblitze sichtbar sind. Hierauf wird die Leistung des Wechselstromgenerators um 20 % abgesenkt. Auf der Bodenplatte bildet sich ein blauer Festkörper. Der Vorgang wird mit dieser Einstellung des Wechselstromgenerators mehrfach wiederholt.

Beispiel 6

5 g hochmethyliertes Pektin werden in 25 ml

$H_2O$ gegeben und so lange geschüttelt bis ein Gel entsteht. Das Gel wird 36 h bei Raumluft stehen gelassen.

5 g Chitosan werden in 25 ml 0,2 N Essigsäure gelöst und stehen gelassen bis keine Luftbläschen mehr sichtbar sind.

Beide Gele werden wie im vorigen Beispiel in jeweils einen der Zuführkanäle 1, 2 einer Vorrichtung gemäß Fig. 1 und Fig. 2 gefüllt. 50 µl des Pektins und 50 µl des Chitosans werden durch die Zuführkanäle in den Mündungsraum 16 der Pipettiervorrichtung eingebracht, wobei ein Wechselstrom von 340 kHz mit einer Leistung von 200 W angelegt wird. Die Auslassöffnung der Pipettiervorrichtung wird dabei an die Bodenplatte angenähert. Auf der Platte bildet sich unter Dampfaustritt ein kleiner bernsteinförmiger Festkörper. Der Vorgang wird zur Fertigung des gewünschten physischen Objekts mehrfach wiederholt.

Beispiel 7

5 g hochmethyliertes Apfelpektin und 5 g Chitosan werden gemischt und in 25 ml 0,2 N Essigsäure aufgenommen, gerührt und bei 6°C 24 Std. gelagert. Das Gemisch wird in den Zuführkanal einer Pipettiervorrichtung gemäß Fig. 3 injiziert und mit der Extrudervorrichtung auf den vorgesehenen Bildungsort 26 befördert. Die Neutralelektrode 19 und die in der Extrudervorrichtung 11 befindliche Elektrode 12 werden mit einem Wechselstrom von 340 kHz und 100 W betrieben. Die Leistung wird schrittweise gesteigert. Unter Dampfaustritt entsteht ein kleiner dunkelbrauner Festkörper.

Beispiel 8

Erstes Polymer: 5 g Carboxymethylstärke DS carboxymethyl 0,4 und 15 g Carboxymethylcellulose werden gemischt und in einem Gemisch aus 100 ml Ethanol und 100 ml 15 % HCl aufgenommen. Die Suspension wird 24 Stunden bei 40 °C gerührt und danach zweimal mit Ethanol und einmal mit Aceton gewaschen, filtriert und anschließend getrocknet. Der getrocknete Feststoff wird in einem Mörser zu einem Pulver zerkleinert und in 300 ml Methanol und 1,5 ml $H_2SO_4$ aufgenommen und 96 Stunden bei 55 °C unter Luftabschluss gerührt. Das Gemisch wird danach zweimal mit Ethanol gewaschen, filtriert und getrocknet und in einem Mörser zerkleinert. Hiervon werden 5 g in 5 mL $H_2O$ aufgenommen und gerührt bis sich ein Gel bildet.

Zweites Polymer: 5 g Chitosan werden in 40 ml 0,2 N Essigsäure aufgenommen und geschüttelt bis sich ein Gel bildet. Das Gel wird mit dem ersten Polymer gemischt und gekühlt aufbewahrt.

Das Gemisch aus dem ersten Polymer und dem zweiten Polymer wird mittels einer Pipettiervorrichtung gemäß Fig. 1 und Fig. 2 zwischen die beiden Elektroden 3 und 6 gepumpt, welche sich beide auf der Pipettiervorrichtung 21 befinden. An die Elektroden wird ein quer zur Flussrichtung des Gemisches ausgerichteter Wechselstrom mit 340 kHz und 140 W angelegt. Danach wird das Polymergemisch mittels einer Extrudervorrichtung 11 auf die Bodenplatte 26 gedrückt und der Wechselstrom von den an der Auslassöffnung 23 der Pipettiervorrichtung 21 bipolar angeordneten Elektroden auf die in der Extrudervorrichtung 11 vorgesehene Elektrode 12 und die im Bereich des vorgesehenen Bildungsorts 26 angeschlossenen Neutralelektrode 19 umgeschaltet. Auf der Bodenplatte 26 entsteht ein dunkelbrauner Feststoff.

Beispiel 9

5 g Carboxymethylstärke werden in 20 ml $H_2O$ gelöst und auf eine mit einer Influenzmaschine negativ aufgeladene rotierende Petrischale aufgetragen. In der Petrischale bildet sich ein durchsichtiger dünner Film. Der Film wird 24 h getrocknet. 100 ml eines Gemischs zu gleichen Teilen bestehend 15%iger Salzsäure und Ethanol wird unter langsamer Rotation vorsichtig auf den Film aufgetropft, 3 Stunden bei 25 °C abdeckt stehen gelassen und danach abzentrifugiert. Der Vorgang wird zweimal wiederholt. Die Schale wird luftdicht verschlossen und mit 200 mL Methanol und 1,5 mL $H_2SO$ gefüllt und 48 Stunden bei 55 °C gehalten. Es bildet sich ein weißliches Gel. Das Gel wird zweimal mit Ethanol und schließlich mit Aceton gewaschen, getrocknet und gekühlt aufbewahrt.

1 mg der Trockensubstanz wird unter Zugabe von $H_2O$ gelöst, mit 1 ml der Genipinlösung aus Beispiel 1 gemischt und bei 4 °C aufbewahrt.

2 g Chitosan werden in 50 mL 0,2 N Essigsäurelösung gelöst und 24 Stunden bei 25 °C stehen gelassen bis keine Luftbläschen mehr sichtbar sind.

Die beiden Polymerkomponenten bestehend aus methylierter Carboxymethylstärke sowie Genipin als Linker einerseits und Chitosan andererseits werden in jeweils einen Zuführkanal der Pipettiervorrichtung gemäß Fig. 1 und 2 gegeben und jeweils zu 5 µl in den jeweiligen Zuführkanal gepumpt. An zwei an der Auslassöffnung 23 der Pipettiervorrichtung angeordnete Elektroden wird ein Wechselstrom von 340 kHz und 40 W angelegt. An ein zweites, versetzt auf der Pipettiervorrichtung befindliches Elektrodenpaar wird einen Wechselstrom von 340 kHz und 100 W angeschlossen und wechselnd zu dem ersten betrieben. Danach werden die Substanzen mit der Extrudervorrichtung auf eine an die Neutralelektrode 19 angeschlossene und mit einen Chitosanfilm belegte Bodenplatte zubewegt, welche als Bildungsort für das zu fügende physische Objekt vorgesehen ist. Der Wechselstrom wird auf die in der Extrudervorrichtung eingebaute Elektrode und die an die Bodenplatte angeschlossenen Neutralelektrode 19 umgeschaltet. Es entsteht ein dunkelblauer Feststoff.

**Patentansprüche**

**1.** Verfahren zur additiven Fertigung eines räumlich ausgebildeten physischen Objekts an einem vorbestimmten Bildungsort (26), bei dem
ein erstes Polymer, dessen Monomereinheiten koppelbare erste reaktive Seitengruppen aufweisen,
und ein zweites Polymer, dessen Monomereinheiten koppelbare zweite reaktive Seitengruppen aufweisen, miteinander zu dem physischen Objekt vernetzt werden,
wobei das erste Polymer und das zweite Polymer mithilfe einer Dosiervorrichtung (17) in ein elektrisches Wechselstromfeld eingebracht werden,
**dadurch gekennzeichnet, dass**
das elektrische Wechselstromfeld zwischen wenigstens einer ersten Elektrode (3, 4, 12), die im Bereich einer Auslassöffnung (23) der Dosiervorrichtung (17) angeordnet ist, und wenigstens einer zweiten Elektrode (5, 6, 19), die beabstandet von der ersten Elektrode (3, 4, 12) im Bereich der Auslassöffnung (23) des Mündungsraums (16) oder im Bereich des vorbestimmten Bildungsortes (26) des physischen Objekts angeordnet ist, erzeugt wird,
wobei die Spannung, die Frequenz, die Stromstärke und die Feldenergiestärke des elektrischen Wechselstromfeldes derart gewählt werden, dass die ersten reaktiven Seitengruppen des ersten Polymers und die zweiten reaktiven Seitengruppen des zweiten Polymers durch eine elektrostriktiv unterstützte chemische Reaktion miteinander verbunden werden.

2. Verfahren nach Anspruch 1, **dadurch gekennzeichnet, dass** das erste und/oder das zweite Polymer ein Polysaccharid, insbesondere ein überwiegend α-1,4-glycosidisch verknüpftes Polysaccharid oder ein überwiegend β-1,4-glycosidisch verknüpftes Polysaccharid, oder ein Protein ist.

3. Verfahren nach einem der Ansprüche 1 oder 2, **dadurch gekennzeichnet, dass** das erste Polymer ein Polysaccharid ist, das Monomere aufweist, deren reaktive Seitengruppen wenigstens eine Aldehydgruppe aufweisen, und das zweite Polymer ein Polysaccharid ist, das Monomere aufweist, deren reaktive Seitengruppen wenigstens eine freie Aminogruppe aufweisen.

4. Verfahren nach Anspruch 3, **dadurch gekennzeichnet, dass** das erste Polymer 2,3-Dialdehyd-Carboxyalkylcellulose ist, wobei die Alkylgruppe 1 bis 4 Kohlenstoffatome aufweist, insbesondere 2,3-Dialdehyd-Carboxymethylcellulose ist und das zweite Polymer bevorzugt ausgewählt ist aus der Gruppe bestehend aus Chitin, Chitosan und Aminoderivaten davon.

5. Verfahren nach einem der vorangehenden Ansprüche, **dadurch gekennzeichnet, dass** weiterhin ein Linker in das elektrische Wechselstromfeld eingebracht wird, der in der Lage ist, die ersten reaktiven Seitengruppen des ersten Polymers und die zweiten reaktiven Seitengruppen des zweiten Polymers miteinander zu koppeln.

6. Verfahren nach einem der vorangehenden Ansprüche, **dadurch gekennzeichnet, dass** ein elektrisches Wechselstromfeld mit einer Frequenz von bis zu 100 GHz, vorzugsweise 200 kHz bis 1 GHz, besonders bevorzugt 200 kHz bis 350 kHz und einer Leistung von 40 bis 100 Watt eingesetzt wird.

7. Verfahren nach einem der vorangehenden Ansprüche, **dadurch gekennzeichnet, dass** die Veränderung der dielektrischen Eigenschaften der Polymere in dem angelegten elektrischen Feld gemessen wird.

8. Vorrichtung zur additiven Fertigung und Applikation eines räumlich ausgebildeten physischen Objekts an einem vorbestimmbaren Bildungsort (26), insbesondere zur Durchführung des Verfahrens nach einem der Ansprüche 1 bis 7, mit
einer räumlich verlagerbaren Dosiervorrichtung (17), die einen Mündungsraum (16) mit einer Auslassöffnung (23) und wenigstens einem Zuführkanal (1, 2) zur dosierbaren Zuführung von Komponenten (31, 32) in den Mündungsraum (16) aufweist,
und einer Wechselstromgeneratoreneinheit (18), an der wenigstens eine erste Elektrode (3, 4, 12) und wenigstens eine zweite Elektrode (5, 6, 19) angeschlossen sind, wobei die Spannung, die Frequenz, die Stromstärke und die Feldenergiestärke jedes von der Wechselstromgeneratoreneinheit (18) erzeugten elektrischen Wechselstromfeldes wählbar sind,
**dadurch gekennzeichnet, dass**
die erste Elektrode (3, 4, 12) im Bereich der Auslassöffnung (23) des Mündungsraums (16) angeordnet ist und die zweite Elektrode (5, 6, 19) beabstandet von der ersten Elektrode (3, 4, 12) im Bereich der Auslassöffnung (23) des Mündungsraums (16) oder im Bereich des vorbestimmten Bildungsorts (26 des physischen Objekts angeordnet ist..

9. Vorrichtung nach Anspruch 8, **dadurch gekennzeichnet, dass** wenigstens zwei Zuführkanäle (1, 2) zur getrennt dosierbaren Zuführung einer ersten fließfähigen Komponente (31) und einer zweiten fließfähigen Komponente (32) in den Mündungsraum (16) vorgesehen sind.

10. Vorrichtung nach einem der Ansprüche 8 oder 9, **dadurch gekennzeichnet, dass** eine zweite Elektrode (19) im Wesentlichen unabhängig von der Dosiervorrichtung (17) räumlich verlagerbar ist.

11. Vorrichtung nach einem der Ansprüche 8 bis 10, **dadurch gekennzeichnet, dass** ein in den Mündungsraum (16) mündender Führungskanal (28) vorgesehen ist.

**12.** Vorrichtung nach einem der Ansprüche 8 bis 11, **dadurch gekennzeichnet dass** eine Extrudervorrichtung (11) verschieblich im Wesentlichen innerhalb des Führungskanals (28) angeordnet ist und in den Mündungsraum (16) ein- und ausfahrbar ist, sodass in dem Mündungsraum (16) vorliegende fließfähige Komponenten (31, 32) in Richtung der Auslassöffnung (23) verlagerbar sind.

**13.** Vorrichtung nach Anspruch 12, **dadurch gekennzeichnet, dass** die Extrudervorrichtung (11) im Bereich ihres in den Mündungsraum (16) weisenden Endes eine weitere erste Elektrode (12) aufweist.

**14.** Vorrichtung nach einem der Ansprüche 8 bis 13, **dadurch gekennzeichnet, dass** eine erste Elektrode (3) und eine zweite Elektrode (6) als Teil einer Messeinheit zur Bestimmung und Überwachung der dielektrischen Eigenschaften der Komponenten in dem elektrischen Wechselstromfeld vorgesehen ist.

**15.** Vorrichtung nach einem der Ansprüche 8 bis 14, **dadurch gekennzeichnet, dass** die Vorrichtung als endoskopisches Arbeitsgerät ausgebildet ist.

**Claims**

**1.** A method for the additive manufacture of a three-dimensional physical object at a pre-determined formation site (26), in which
a first polymer, whose monomer units comprise first reactive side groups amenable to coupling,
and a second polymer, whose monomer units comprise second reactive side groups amenable to coupling, are crosslinked with one another to give the physical object,
wherein the first polymer and the second polymer are introduced with the aid of a metering device (17) into an alternating-current electric field,
**characterised in that**
the alternating-current electric field is generated between at least one first electrode (3, 4, 12), which is arranged in the region of an outlet aperture (23) of the metering device (17), and at least one second electrode (5, 6, 19), which is arranged at a distance from the first electrode (3, 4, 12) in the region of the outlet aperture (23) of the orifice space (16) or in the region of the pre-determined formation site (26) of the physical object,
wherein the voltage, the frequency, the current intensity and the field energy intensity of the alternating-current electric field are selected in such a way that the first reactive side groups of the first polymer and the second reactive side groups of the second polymer become bonded to one another via an electrostrictively assisted chemical reaction.

**2.** The method according to claim 1, **characterised in that** the first and/or the second polymer is a polysaccharide, in particular a predominantly $\alpha$-1,4-glycosidically linked polysaccharide or a predominantly $\beta$-1,4-glycosidically linked polysaccharide, or a protein.

**3.** The method according to one of claims 1 or 2, **characterised in that** the first polymer is a polysaccharide comprising monomers whose reactive side groups comprise at least one aldehyde group, and the second polymer is a polysaccharide comprising monomers whose reactive side groups comprise at least one free amino group.

**4.** The method according to claim 3, **characterised in that** the first polymer is 2,3-dialdehyde carboxyalkyl cellulose, wherein the alkyl group comprises 1 to 4 carbon atoms, and is in particular 2,3-dialdehyde carboxymethyl cellulose, and the second polymer is preferably selected from the group consisting of chitin, chitosan and amino derivatives thereof.

**5.** The method according to one of the preceding claims, **characterised in that** a linker is furthermore introduced into the alternating-current electric field, said linker being capable of coupling the first reactive side groups of the first polymer and the second reactive side groups of the second polymer with one another.

**6.** The method according to one of the preceding claims, **characterised in that** an alternating-current electric field with a frequency of up to 100 GHz, preferably 200 kHz to 1 GHz, particularly preferably 200 kHz to 350 kHz, and a power output of 40 to 100 watts is employed.

**7.** The method according to one of the preceding claims, **characterised in that** the change in the dielectric properties of the polymers in the applied electric field is measured.

**8.** A device for the additive manufacture and application of a three-dimensional physical object at a predetermined formation site (26), in particular for implementing the method according to one of claims 1 to 7, with a spatially displaceable metering device (17), comprising an orifice space (16) with an outlet aperture (23) and at least one supply channel (1, 2) for the meterable supply of components (31, 32) into the orifice space (16),
and an alternating-current generator unit (18), to which at least one first electrode (3, 4, 12) and at least one second electrode (5, 6, 19) are connected, wherein the voltage, the frequency, the current intensity and the field energy intensity of each alternating-current electric field generated by the alternating-current generator unit (18) are selectable, **characterised in that** the first electrode (3, 4, 12) is arranged in the region of the outlet aperture (23) of the orifice space (16) and the second electrode (5, 6, 19) is arranged at a distance from the first electrode (3, 4, 12) in the region of the outlet aperture (23) of the orifice space (16) or in the region of the predetermined formation site (26) of the physical object.

9. The device according to claim 8, **characterised in that** at least two supply channels (1, 2) are provided for the separately meterable supply of a first flowable component (31) and a second flowable component (32) into the orifice space (16).

10. The device according to one of claims 8 or 9, **characterised in that** a second electrode (19) is spatially displaceable substantially independently of the metering device (17).

11. The device according to one of claims 8 to 10, **characterised in that** a guide channel (28) leading into the orifice space (16) is provided.

12. The device according to one of claims 8 to 11, **characterised in that** an extruder device (11) is slidably arranged substantially inside the guide channel (28) and can be moved in and out of the orifice space (16) such that flowable components (31, 32) present in the orifice space (16) are displaceable towards the outlet aperture (23).

13. The device according to claim 12, **characterised in that** the extruder device (11) comprises a further first electrode (12) in the region of its end facing into the orifice space (16).

14. The device according to one of claims 8 to 13, **characterised in that** a first electrode (3) and a second electrode (6) are provided as part of a measuring unit for determining and monitoring the dielectric properties of the components in the alternating-current electric field.

15. The device according to one of claims 8 to 14, **characterised in that** the device is configured as an endoscopic working apparatus.

## Revendications

1. Procédé de fabrication additive d'un objet physique présentant une structure spatiale, en un site de formation (26) prédéfini, dans lequel un premier polymère, dont les motifs monomères comportent des premiers groupes latéraux réactifs pouvant être couplés, et un second polymère, dont les motifs monomères comportent des seconds groupes latéraux réactifs pouvant être couplés, sont réticulés l'un avec l'autre en l'objet physique, le premier polymère et le second polymère étant introduits dans un champ électrique de courant alternatif à l'aide d'un dispositif de dosage (17), **caractérisé en ce que** le champ électrique de courant alternatif est généré entre au moins une première électrode (3, 4, 12), qui est disposée dans la zone d'un orifice de sortie (23) du dispositif de dosage (17), et au moins une seconde électrode (5, 6, 19), qui est disposée à distance de la première électrode (3, 4, 12) dans la zone de l'orifice de sortie (23) de l'espace d'embouchure (16) ou dans la zone du site de formation (26) prédéfini de l'objet physique, la tension, la fréquence, l'intensité de courant et la force de l'énergie de champ du champ électrique de courant alternatif étant choisies de telle manière que les premiers groupes latéraux réactifs du premier polymère et les seconds groupes latéraux réactifs du second polymère sont reliés les uns aux autres par une réaction chimique assistée par électrostriction.

2. Procédé selon la revendication 1, **caractérisé en ce que** le premier et/ou le second polymère est un polysaccharide, en particulier un polysaccharide lié principalement par une liaison glycosidique en α-1,4 ou un polysaccharide lié principalement par une liaison glycosidique en β-1,4, ou une protéine.

3. Procédé selon l'une des revendications 1 ou 2, **caractérisé en ce que** le premier polymère est un polysaccharide, qui comporte des monomères dont les groupes latéraux réactifs comportent au moins un groupe aldéhyde, et le second polymère est un polysaccharide, qui comporte des monomères dont les groupes latéraux réactifs comportent au moins un groupe amine libre.

4. Procédé selon la revendication 3, **caractérisé en ce que** le premier polymère est de la 2,3-dialdéhyde-carboxyalkylcellulose, le groupe alkyle comportant 1 à 4 atomes de carbone, en particulier de la 2,3-dialdéhyde-carboxyméthylcellulose, et le second polymère est choisi de préférence dans le groupe constitué de la chitine, du chitosane et de dérivés d'aminés de ceux-ci.

5. Procédé selon l'une des revendications précédentes, **caractérisé en ce qu'**un coupleur est en outre introduit dans le champ électrique de courant alternatif, qui est apte à coupler les uns aux autres les premiers groupes latéraux réactifs du premier polymère et les seconds groupes latéraux réactifs du second polymère.

6. Procédé selon l'une des revendications précédentes, **caractérisé en ce qu'**un champ électrique de courant alternatif avec une fréquence allant jusqu'à 100 GHz, de préférence de 200 kHz à 1 Ghz, de manière particulièrement préférée de 200 kHz à 350 kHz et une puissance de 40 à 100 watts est utilisé.

7. Procédé selon l'une des revendications précédentes, **caractérisé en ce que** la modification des propriétés diélectriques des polymères dans le champ électrique appliqué est mesurée.

8. Dispositif de fabrication additive et application d'un objet physique présentant une structure spatiale, en un site de formation (26) pouvant être prédéfini, en particulier pour exécuter le procédé selon l'une des revendications 1 à 7, comprenant un dispositif de dosage (17) pouvant être déplacé dans l'espace, qui comporte un espace d'embouchure (16) avec un orifice de sortie (23) et au moins un conduit d'alimentation (1, 2) pour l'alimentation pouvant être dosée de composants (31, 32) dans l'espace d'embouchure (16), et une unité de générateurs de courant alternatif (18), à laquelle sont raccordées au moins une première électrode (3, 4, 12) et au moins une seconde électrode (5, 6, 19), la tension, la fréquence, l'intensité de courant et la force d'énergie de champ de chaque champ électrique de courant alternatif généré par l'unité de générateurs de courant alternatif (18) pouvant être choisies, **caractérisé en ce que** la première électrode (3, 4, 12) est disposée dans la zone de l'orifice de sortie (23) de l'espace d'embouchure (16) et la seconde électrode (5, 6, 19) est disposée à distance de la première électrode (3, 4, 12) dans la zone de l'orifice de sortie (23) de l'espace d'embouchure (16) ou dans la zone du site de formation (26) prédéfini de l'objet physique.

9. Dispositif selon la revendication 8, **caractérisé en ce qu'**au moins deux conduits d'alimentation (1, 2) sont prévus pour l'alimentation pouvant être dosée séparément d'un premier composant (31) à écoulement libre et d'un second composant (32) à écoulement libre dans l'espace d'embouchure (16).

10. Dispositif selon l'une des revendications 8 ou 9, **caractérisé en ce qu'**une seconde électrode (19) peut être déplacée dans l'espace essentiellement indépendamment du dispositif de dosage (17).

11. Dispositif selon l'une des revendications 8 à 10, **caractérisé en ce qu'**un conduit de guidage (28) débouchant sur l'espace d'embouchure (16) est prévu.

12. Dispositif selon l'une des revendications 8 à 11, **caractérisé en ce qu'**un dispositif d'extrusion (11) est disposé mobile essentiellement dans le conduit de guidage (28) et peut être entré dans l'espace d'embouchure (16) et sorti de celui-ci, de telle sorte que des composants (31, 32) à écoulement libre présents dans l'espace d'embouchure (16) peuvent être déplacés en direction de l'orifice de sortie (23) .

13. Dispositif selon la revendication 12, **caractérisé en ce que** le dispositif d'extrusion (11) comporte, dans la zone de son extrémité orientée vers l'espace d'embouchure (16), une autre première électrode (12).

14. Dispositif selon l'une des revendications 8 à 13, **caractérisé en ce qu'**une première électrode (3) et une seconde électrode (6) sont prévues comme parties d'une unité de mesure pour déterminer et surveiller les propriétés diélectriques des composants dans le champ électrique de courant alternatif.

15. Dispositif selon l'une des revendications 8 à 14, **caractérisé en ce que** le dispositif est réalisé sous la forme d'un outil endoscopique.

**Fig. 1**

**Fig. 1A**

**Fig. 1B**

**Fig. 1C**

**Fig. 2**

## Fig. 3A

# Fig. 3B

**Fig. 3C**

**Fig. 4**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20040251581 A1
- US 20110136162 A1
- US 20120018924 A1
- US 4124758 A

**In der Beschreibung aufgeführte Nicht-Patentliteratur**

- **LINDH. J. ; CARLSON, O.C. ; STROMME, M. ; MIHRANAN, A.** Convenient One-Pot Formation of 2,3-Dialdehyd-Cellulose Beads via Peroxidate Oxidation of Cellulose in Water. *Biomacromolecules,* 2014, vol. 15, 1928-1932